# EUROPEAN PATENT APPLICATION

(11) **EP 4 410 837 A1**
(43) Date of publication of application: **07.08.2024**
(21) Application number: 22875065.9
(22) Date of filing: 29.09.2022
(51) Int. Cl.: C07K 16/28, C07K 16/46, A61K 39/395, A61K 38/00, C12N 15/85, C12N 15/13, C12N 15/63, A61P 35/00, A61P 35/02

(54) **ANTI-LAG3 BISPECIFIC ANTIBODY, PHARMACEUTICAL COMPOSITION AND USE**

(30) Priority: 29.09.2021 CN 202111149114
(71) Applicant: Akeso Huike (Shanghai) Co. Ltd., Shanghai 200137 (CN)
(72) Inventor: ZHANG, Peng, Zhongshan, Guangdong 528437 (CN); LI, Baiyong, Zhongshan, Guangdong 528437 (CN); XIA, Yu, Zhongshan, Guangdong 528437 (CN); WANG, Zhongmin, Zhongshan, Guangdong 528437 (CN)
(74) Representative: Berggren Oy
(86) International application number: PCT/CN2022/122556
(87) International publication number: WO 2023/051683

(57) **Abstract**

Provided are an anti-LAG3 antibody bispecific antibody, a pharmaceutical composition thereof and a use thereof, which belong to the field of biomedicine. Specifically, the bispecific antibody comprises a first protein functional domain and a second protein functional domain, wherein the first protein functional domain targets LAG3, and the second protein functional domain targets a target that is different from LAG3, the first protein functional domain is an anti-LAG3 antibody or an antigen-binding fragment thereof and contains a heavy chain variable region and a light chain variable region, the heavy chain variable region contains HCDR1-HCDR3 the amino acid sequences of which are respectively represented by SEQ ID NOs: 5-7. In addition, the light chain variable region contains LCDR1-LCDR3 the amino acid sequences of which are respectively represented by SEQ ID NOs: 8-10. The bispecific antibody has excellent affinity and specificity, and has good application prospects.

## Description

### TECHNICAL FIELD

The present invention belongs to the field of biomedicine, and relates to an anti-LAG3 bispecific antibody, and a pharmaceutical composition thereof and use thereof. Specifically, the bispecific antibody is an anti-LAG3/anti-PD-1 bispecific antibody.

### BACKGROUND

Tumor, especially a malignant tumor, is a serious health-threatening disease in the world today, and it is the second leading cause of death among various diseases. In recent years, the incidence of the disease has been increasing remarkably. The malignant tumor is characterized by poor treatment response, high late metastasis rate, and poor prognosis. Although conventional treatment methods (such as radiotherapy, chemotherapy, and surgical treatment) adopted clinically at present alleviate the pain to a great extent and prolong the survival time, the methods have great limitations, and it is difficult to further improve their efficacy.

Lymphocyte-activation gene 3 (LAG3), namely CD223, is a type I transmembrane protein composed of 498 amino acids and is a member of the immunoglobulin superfamily (IgSF). LAG3 is mainly expressed in activated CD4⁺ T cells and CD8⁺ T cells. Additionally, in cells such as natural killer (NK) cells, B cells, regulatory T cells (Tregs), and plasmacytoid dendritic cells (pDCs), LAG3 is also expressed. (Ruffo Elisa, Wu Richard C, Bruno Tullia C et al., Lymphocyte-activation gene 3 (LAG3): The next immune checkpoint receptor. [J].Semin Immunol, 2019, 42: 101305.).

The LAG3 molecule gene is located on human chromosome 12 (20p13.3), adjacent to the CD4 molecule gene, and both have the same exons and introns. The LAG3 molecule and the CD4 molecule have a high degree of structural similarity, although the amino acid sequence homology between the two is only about 20%. Major histocompatibility complex class II (MHC II) molecules, liver sinusoidal endothelial cell lectin (LSECtin) molecules, and galectin-3 molecules are related ligands for the LAG3 molecule. The MHC class II molecules are the main ligands for LAG3. The affinity (Kd: 60 nmol·L⁻¹) of LAG3 molecules for the MHC class II molecules is 100 times that of CD4 molecules, indicating that the LAG3 molecules can effectively compete with the CD4 molecules for binding to the MHC class II molecules and inhibit T cell activation.

In the tumor microenvironment, the expression of the immunosuppressive molecule LAG3 can be detected 24 hours after T cell activation, which then leads to T cell dysfunction or apoptosis. The LAG3 molecule, through its D1 domain (which contains one proline-rich loop structure), forms a dimer molecule to specifically bind to the MHC class II molecule in the first signaling axis of CD4⁺ T cell activation, "CD3-TCR-MHCII", so that on one hand, a signal transduction pathway for T cell activation is blocked, and on the other hand, an intracellular segment of the LAG3 molecule (HIEELE motif) generates an immunosuppressive signal to down-regulate the activity of CD4⁺ T cells. The LAG3 molecule can promote the differentiation of Treg cells, participate in downstream signaling of signal transducer and activator of transcription 5, and thus enhance the inhibitory effect of Treg cells, which is one of the mechanisms by which tumors escape from killing by the immune system (Andrews Lawrence P, Marciscano Ariel E, Drake Charles G, et al., LAG3 (CD223) as a cancer immunotherapy target. [J]. Immunol Rev, 2017, 276: 80-96.).

Multiple studies have shown that LAG3 is overexpressed in tumor-infiltrating CD8⁺ T cells of various malignant tumors. For example, in ovarian cancer, tumor-infiltrating New York esophageal squamous cell carcinoma 1 (NY-ESO-1) antigen-specific CD8⁺ T cells express high levels of PD-1 and LAG3, and have a reduced ability to produce IFN-y and TNF-a, thereby leading to lymphocyte inactivation. Galectin-3 and LSECtin interact primarily with LAG3 to regulate the activation and function of CD8⁺ T cells. In addition, melanoma antigen-specific T cells isolated from patients with metastatic melanoma exhibit a significant upregulation in the expression of LAG3 and other immune checkpoint molecules CTLA-4 and TIM-3. (Liu Hao, Li Xinying, Luo Longlong, et al., Research advances in biological function of lymphocyte activation gene-3 (LAG-3) molecule and clinical application of antibody drugs targeting LAG-3 [J]. Chinese Journal of Pharmacology and Toxicology, 2019, 33(01): 70-78.).

Currently, a plurality of LAG3 antibody medicaments have entered the clinical research stages, among which Bristol Myers Squibb's Relatlimab has the fastest progress, with 10 clinical studies underway. The vast majority of these studies involve the combination therapy of Relatlimab with nivolumab, used for the treatment of tumors such as hematological malignancies, melanoma, glioblastoma, renal cell carcinoma, non-small cell lung cancer, and the like.

The transmembrane receptor PD-1 (programmed cell death protein 1) is a member of the CD28 family, and is expressed in activated T cells, B cells and myeloid cells. The receptors of PD-1, PDL1 and PDL2, are members of the B7 superfamily. PDL1 is expressed in a variety of cells including T cells, B cells, endothelial cells and epithelial cells, and PDL2 is expressed only in antigen-presenting cells such as dendritic cells and macrophages.

PD-1 plays a very important role in down-regulating the activation of T cells, and the PD-1-mediated down-regulation of T cells is one of the important mechanisms for tumor immune escape. PD-L1 expressed on the surface of tumors can bind to PD-1 on the surface of immune cells, thereby inhibiting the killing of tumor tissues by the immune cells through the PD-1/PD-L1 signaling pathway, and tumors with high expression of PD-L1 are associated with cancers that are difficult to detect (Hamanishi et al., Proc. Natl. Acad. Sci. USA, 2007; 104: 3360-5). An effective way to antagonize PD-1 and thus inhibit the PD-1/PD-L1 signaling pathway is the *in-vivo* injection of anti-PD-1 antibody.

Due to the broad anti-tumor prospects and surprising efficacy of PD-1 antibodies, antibodies targeting the PD-1 pathway will bring about breakthroughs in the treatment of a variety of tumors: non-small cell lung cancer, renal cell carcinoma, ovarian cancer, and melanoma (Homet M. B., Parisi G., et al., Anti-PD-1 therapy in melanoma. Semin Oncol, 2015 Jun; 42(3): 466-473), and hematological tumor and anemia (Held SA, Heine A, et al., Advances in immunotherapy of chronic myeloid leukemia CML. Curr Cancer Drug Targets, 2013 Sep; 13(7): 768-74).

Bifunctional antibodies, also known as bispecific antibodies, are specific antibody medicaments that target two different antigens simultaneously, and can be produced by immunosorting and purification, or can be obtained by genetic engineering. The genetic engineering has flexibility in aspects of binding site optimization, synthetic form, yield, and the like, thus having certain advantages. Currently, the bispecific antibody has been demonstrated to exist in over 45 forms (Müller D, Kontermann RE. Bispecific antibodies for cancer immunotherapy: Current perspectives. BioDrugs 2010; 24: 89-98). The IgG-ScFv form, namely the Morrison form (Coloma MJ, Morrison SL. Design and production of novel tetravalent bispecific antibodies. Nat Biotechnol. Nature Biotechnology, 1997; 15: 159-163), has been demonstrated to be an ideal form of the bifunctional antibody due to its similarity to the naturally existing IgG form and advantages in antibody engineering, expression and purification (Miller BR, Demarest SJ, et al., Stability engineering of scFvs for the development of bispecific and multivalent antibodies. Protein Eng Des Sel, 2010; 23: 549-57; Fitzgerald J, Lugovskoy A. Rational engineering of antibody therapeutics targeting multiple oncogene pathways. MAbs, 2011; 3: 299-309).

There is currently a need to develop a novel anti-LAG3 antibody and a bifunctional antibody medicament that targets PD-1 and LAG3 simultaneously.

### SUMMARY

Through intensive studies and creative efforts, the inventors have obtained an anti-LAG3 antibody, and based on this, have developed an anti-LAG3/anti-PD-1 bispecific antibody. The inventors have surprisingly found that the anti-LAG3 antibody of the present invention (also referred to as the antibody or the antibody of the present invention for short) and the anti-LAG3/anti-PD-1 bispecific antibody of the present invention (also referred to as the bispecific antibody or the bispecific antibody of the present invention for short) have superior affinity and/or specificity, and are even superior in one or more aspects compared to positive control antibodies (e.g., Nivolumab, Pembrolizumab, Relatlimab, and the like). The present invention is detailed below.

One aspect of the present invention relates to an anti-LAG3 antibody or an antigen-binding fragment thereof, comprising a heavy chain variable region and a light chain variable region, wherein
the heavy chain variable region comprises HCDR1-HCDR3 having amino acid sequences set forth in SEQ ID NOs: 5-7, respectively, and the light chain variable region comprises LCDR1-LCDR3 having amino acid sequences set forth in SEQ ID NOs: 8-10, respectively;
the heavy chain variable region comprises HCDR1-HCDR3 having amino acid sequences set forth in SEQ ID NOs: 5-7, respectively, and the light chain variable region comprises LCDR1-LCDR3 having amino acid sequences set forth in SEQ ID NO: 8, SEQ ID NO: 46 and SEQ ID NO: 47, respectively;
   or
the heavy chain variable region comprises HCDR1-HCDR3 having amino acid sequences set forth in SEQ ID NOs: 5-7, respectively, and the light chain variable region comprises LCDR1-LCDR3 having amino acid sequences set forth in SEQ ID NO: 48, SEQ ID NO: 46 and SEQ ID NO: 10, respectively.

In some embodiments of the present invention, the antibody or the antigen-binding fragment thereof is provided, wherein
the heavy chain variable region of the antibody has an amino acid sequence set forth in SEQ ID NO: 2, and the light chain variable region of the antibody has an amino acid sequence set forth in SEQ ID NO: 4;
the heavy chain variable region of the antibody has an amino acid sequence set forth in SEQ ID NO: 2, and the light chain variable region of the antibody has an amino acid sequence set forth in SEQ ID NO: 42;
   or
the heavy chain variable region of the antibody has an amino acid sequence set forth in SEQ ID NO: 2, and the light chain variable region of the antibody has an amino acid sequence set forth in SEQ ID NO: 44.

In some embodiments of the present invention, the antibody or the antigen-binding fragment thereof is provided, wherein the antibody or the antigen-binding fragment thereof is selected from a Fab, a Fab', an F(ab')₂, an Fd, an Fv, a dAb, a complementarity determining region fragment, a single chain fragment variable, a humanized antibody, a chimeric antibody, and a diabody.

In some embodiments of the present invention, the antibody or the antigen-binding fragment thereof is provided, wherein the antibody binds to human LAG3-mFc with an EC₅₀ of less than 0.2 nM, such as less than 0.15 nM, less than 0.1 nM, less than 0.08 nM, 0.06 nM, or less than 0.05 nM, or less; preferably, the EC₅₀ value is determined by indirect ELISA.

In some embodiments of the present invention, the antibody or the antigen-binding fragment thereof is provided, wherein
the antibody comprises a non-CDR region derived from a species other than murine, such as from a human antibody.

In some embodiments of the present invention, the antibody or the antigen-binding fragment thereof is provided, wherein
the antibody comprises a constant region derived from a human antibody;
preferably, the constant region of the antibody is selected from constant regions of human IgG1, IgG2, IgG3 or IgG4.

In some embodiments of the present invention, the antibody or the antigen-binding fragment thereof is provided, wherein
a heavy chain constant region of the anti-LAG3 antibody is Ig gamma-1 chain C region (e.g., as set forth in SEQ ID NO: 39) or Ig gamma-4 chain C region (e.g., as set forth in SEQ ID NO: 45), and a light chain constant region of the anti-LAG3 antibody is Ig kappa chain C region (e.g., as set forth in SEQ ID NO: 40).

In some embodiments of the present invention, the antibody or the antigen-binding fragment thereof is provided, wherein
the antibody is of human IgG1 subtype,
wherein according to the EU numbering system, the heavy chain constant region of the antibody has the following mutations:
   L234A and L235A;
   L234A and G237A;
   L235A and G237A;
      or
   L234A, L235A and G237A;
   preferably, the antibody comprises a heavy chain having an amino acid sequence set forth in SEQ ID NO: 11, and a light chain having an amino acid sequence set forth in SEQ ID NO: 12.

In some embodiments of the present invention, the antibody or the antigen-binding fragment thereof is provided, wherein
the antibody is of human IgG4 subtype,
wherein according to the EU numbering system, the heavy chain constant region of the antibody has the following mutations:
   F234A and L235A;
   F234A and G237A;
   L235A and G237A;
      or
   F234A, L235A and G237A;
   preferably, the antibody comprises a heavy chain having an amino acid sequence set forth in SEQ ID NO: 13, and a light chain having an amino acid sequence set forth in SEQ ID NO: 12.

In some embodiments of the present invention, the anti-LAG3 antibody is a monoclonal antibody.

In some embodiments of the present invention, the anti-LAG3 antibody is in an immunoglobulin form.

In some embodiments of the present invention, the anti-LAG3 antibody is a single chain fragment variable.

Another aspect of the present invention relates to an antibody-drug conjugate (ADC), comprising an antibody or an antigen-binding fragment thereof and a small molecule drug, wherein the antibody or the antigen-binding fragment thereof is the anti-LAG3 antibody or the antigen-binding fragment thereof according to any embodiment of the present invention; preferably, the small molecule drug is a small molecule cytotoxic drug; and more preferably, the small molecule drug is an anti-tumor chemotherapeutic drug.

The chemotherapeutic drug may be a conventional anti-tumor chemotherapeutic drug, such as an alkylating agent, an antimetabolite, an anti-tumor antibiotic, a plant-based anticancer agent, a hormone, and an immunological agent.

In one or more embodiments of the present invention, the antibody-drug conjugate is provided, wherein the antibody or the antigen-binding fragment thereof is linked to the small molecule drug via a linker; the linker may be one known to those skilled in the art, for example, a hydrazone bond, a disulfide bond, or a peptide bond.

In one or more embodiments of the present invention, the antibody-drug conjugate is provided, wherein the molar ratio of the antibody or the antigen-binding fragment thereof to the small molecule drug is 1:(2-4), e.g., 1:2, 1:3, or 1:4.

Yet another aspect of the present invention relates to a bispecific antibody comprising a first protein functional region and a second protein functional region, wherein
the first protein functional region targets LAG3, and
the second protein functional region targets a target other than LAG3 (e.g., PD-1),
wherein the first protein functional region is the antibody or the antigen-binding fragment thereof according to any embodiment of the present invention;
preferably, the bispecific antibody is in an IgG-scFv form;
preferably, the first protein functional region is the antibody according to any embodiment of the present invention, and the second protein functional region is a single chain fragment variable; or
preferably, the first protein functional region is a single chain fragment variable, and the second protein functional region is the antibody according to any embodiment of the present invention.

The bispecific antibody of the present invention is an anti-LAG3/anti-PD-1 bispecific antibody.

In some embodiments of the present invention, the bispecific antibody is provided, wherein the first protein functional region and the second protein functional region are linked directly or via a linker fragment;
preferably, the linker fragment is (GGGGS)m, m being a positive integer such as 1, 2, 3, 4, 5, or 6; or
preferably, the linker fragment is (GGGGS)nG, n being a positive integer such as 1, 2, 3, 4, 5, or 6.

In some embodiments of the present invention, the bispecific antibody is provided, wherein the numbers of the first protein functional region and the second protein functional region are each independently 1, 2, or more.

In some embodiments of the present invention, the bispecific antibody is provided, wherein the single chain fragment variable is linked to the C-terminus of the heavy chain of the antibody.

In some embodiments of the present invention, the bispecific antibody is provided, comprising:
a first protein functional region targeting LAG3, and
a second protein functional region targeting PD-1,
wherein
the first protein functional region is the anti-LAG3 antibody according to any embodiment of the present invention, and the anti-LAG3 antibody is in an immunoglobulin form;
the second protein functional region is an anti-PD-1 single chain fragment variable.

In some embodiments of the present invention, the bispecific antibody is provided, wherein the anti-PD-1 single chain fragment variable comprises a heavy chain variable region and a light chain variable region, wherein
the heavy chain variable region comprises HCDR1-HCDR3 having amino acid sequences set forth in SEQ ID NOs: 26-28, respectively; and
the light chain variable region comprises LCDR1-LCDR3 having amino acid sequences set forth in SEQ ID NOs: 29-31, respectively.

In some embodiments of the present invention, the bispecific antibody is provided, wherein in the anti-PD-1 single chain fragment variable,
the heavy chain variable region has an amino acid sequence set forth in SEQ ID NO: 15, and
the light chain variable region has an amino acid sequence set forth in SEQ ID NO: 17; or
the heavy chain variable region has an amino acid sequence set forth in SEQ ID NO: 19, and
the light chain variable region has an amino acid sequence set forth in SEQ ID NO: 21 or SEQ ID NO: 38.

In some embodiments of the present invention, the bispecific antibody is provided, wherein the heavy chain variable region and the light chain variable region in the anti-PD-1 single chain fragment variable are linked directly or via a linker fragment;
preferably, the linker fragment is (GGGGS)m, m being a positive integer such as 1, 2, 3, 4, 5, or 6; or
preferably, the linker fragment is (GGGGS)nG, n being a positive integer such as 1, 2, 3, 4, 5, or 6.

In some embodiments of the present invention, the bispecific antibody is provided, wherein the bispecific antibody comprises:
a first protein functional region targeting LAG3, and
a second protein functional region targeting PD-1;
the number of the first protein functional region is 1, and the number of the second protein functional region is 2;
wherein the first protein functional region is an immunoglobulin, and the second protein functional region is a single chain fragment variable;
the immunoglobulin comprises a heavy chain having an amino acid sequence set forth in SEQ ID NO: 11 or SEQ ID NO: 13, and a light chain having an amino acid sequence set forth in SEQ ID NO: 12;
the single chain fragment variable comprises a heavy chain variable region having an amino acid sequence set forth in SEQ ID NO: 19, and a light chain variable region having an amino acid sequence set forth in SEQ ID NO: 21 or SEQ ID NO: 38;
the single chain fragment variable is linked to the C termini of two heavy chains of the immunoglobulin;
the first protein functional region is linked to the second protein functional region via a first linker fragment; the heavy chain variable region of the single chain fragment variable is linked to the light chain variable region of the single chain fragment variable via a second linker fragment; the first linker fragment and the second linker fragment are identical or different;
preferably, the first linker fragment and the second linker fragment each have an amino acid sequence independently selected from SEQ ID NOs: 35-37;
preferably, amino acid sequences of the first linker fragment and the second linker fragment are set forth in SEQ ID NO: 36.

In some embodiments of the present invention, the bispecific antibody is provided, comprising:
a first protein functional region targeting LAG3, and
a second protein functional region targeting PD-1,
wherein the first protein functional region is an anti-LAG3 single chain fragment variable,
the second protein functional region is an anti-PD-1 antibody, and the anti-PD-1 antibody is in an immunoglobulin form,
wherein the anti-LAG3 single chain fragment variable comprises a heavy chain variable region and a light chain variable region, wherein
the heavy chain variable region comprises HCDR1-HCDR3 having amino acid sequences set forth in SEQ ID NOs: 5-7, respectively; and
the light chain variable region comprises LCDR1-LCDR3 having amino acid sequences set forth in SEQ ID NOs: 8-10, respectively.

In some embodiments of the present invention, the bispecific antibody is provided, wherein in the anti-LAG3 single chain fragment variable,
the heavy chain variable region has an amino acid sequence set forth in SEQ ID NO: 2, and
the light chain variable region has an amino acid sequence set forth in SEQ ID NO: 4;
the heavy chain variable region has an amino acid sequence set forth in SEQ ID NO: 2, and
the light chain variable region has an amino acid sequence set forth in SEQ ID NO: 42; or
the heavy chain variable region has an amino acid sequence set forth in SEQ ID NO: 2, and
the light chain variable region has an amino acid sequence set forth in SEQ ID NO: 44.

In some embodiments of the present invention, the bispecific antibody is provided, wherein
the heavy chain variable region and the light chain variable region in the anti-LAG3 single chain fragment variable are linked directly or via a linker fragment;
preferably, the linker fragment is (GGGGS)m, m being a positive integer such as 1, 2, 3, 4, 5, or 6; or
preferably, the linker fragment is (GGGGS)nG, n being a positive integer such as 1, 2, 3, 4, 5, or 6.

In some embodiments of the present invention, the bispecific antibody is provided, wherein the anti-PD-1 antibody comprises a heavy chain variable region and a light chain variable region, wherein
the heavy chain variable region comprises HCDR1-HCDR3 having amino acid sequences set forth in SEQ ID NOs: 26-28, respectively; and
the light chain variable region comprises LCDR1-LCDR3 having amino acid sequences set forth in SEQ ID NOs: 29-31, respectively.

In some embodiments of the present invention, the bispecific antibody is provided, wherein in the anti-PD-1 antibody,
the heavy chain variable region has an amino acid sequence set forth in SEQ ID NO: 15, and
the light chain variable region has an amino acid sequence set forth in SEQ ID NO: 17; or
the heavy chain variable region has an amino acid sequence set forth in SEQ ID NO: 19, and
the light chain variable region has an amino acid sequence set forth in SEQ ID NO: 21 or SEQ ID NO: 38.

In some embodiments of the present invention, the bispecific antibody is provided, wherein a heavy chain constant region of the anti-PD-1 antibody is Ig gamma-1 chain C region (e.g., as set forth in SEQ ID NO: 39) or Ig gamma-4 chain C region (e.g., as set forth in SEQ ID NO: 45), and a light chain constant region of the anti-PD-1 antibody is Ig kappa chain C region (e.g., as set forth in SEQ ID NO: 40).

In some embodiments of the present invention, the bispecific antibody is provided, wherein
the anti-PD-1 antibody is of human IgG1 subtype,
wherein according to the EU numbering system, the anti-PD-1 antibody has the following mutations:
   L234A and L235A;
   L234A and G237A;
   L235A and G237A;
      or
   L234A, L235A and G237A;
   preferably, the anti-PD-1 antibody comprises a heavy chain having an amino acid sequence set forth in SEQ ID NO: 34, and a light chain having an amino acid sequence set forth in SEQ ID NO: 25.

In some embodiments of the present invention, the bispecific antibody is provided, wherein
the anti-PD-1 antibody is of human IgG4 subtype,
wherein according to the EU numbering system, the anti-PD-1 antibody has the following mutations:
   F234A and L235A;
   F234A and G237A;
   L235A and G237A;
      or
   F234A, L235A and G237A;
   preferably, the anti-PD-1 antibody comprises a heavy chain having an amino acid sequence set forth in SEQ ID NO: 32, and a light chain having an amino acid sequence set forth in SEQ ID NO: 25.

In some embodiments of the present invention, the bispecific antibody is provided, wherein the bispecific antibody comprises:
a first protein functional region targeting LAG3, and
a second protein functional region targeting PD-1;
the number of the first protein functional region is 1, and the number of the second protein functional region is 2;
wherein the first protein functional region is a single chain fragment variable, and the second protein functional region is an immunoglobulin;
the single chain fragment variable comprises a heavy chain variable region having an amino acid sequence set forth in SEQ ID NO: 2, and a light chain variable region having an amino acid sequence set forth in SEQ ID NO: 4;
the immunoglobulin comprises a heavy chain having an amino acid sequence set forth in SEQ ID NO: 34 or SEQ ID NO: 32, and a light chain having an amino acid sequence set forth in SEQ ID NO: 25;
the single chain fragment variable is linked to the C termini of two heavy chains of the immunoglobulin;
the first protein functional region is linked to the second protein functional region via a first linker fragment; the heavy chain variable region of the single chain fragment variable is linked to the light chain variable region of the single chain fragment variable via a second linker fragment; the first linker fragment and the second linker fragment are identical or different;
preferably, the first linker fragment and the second linker fragment each have an amino acid sequence independently selected from SEQ ID NOs: 35-37;
preferably, amino acid sequences of the first linker fragment and the second linker fragment are set forth in SEQ ID NO: 36.

In some embodiments of the present invention, the bispecific antibody is provided, wherein one immunoglobulin molecule is linked to two single chain fragment variable molecules; preferably, the two single chain fragment variable molecules are identical.

Yet another aspect of the present invention relates to an isolated nucleic acid molecule encoding the anti-LAG3 antibody according to any embodiment of the present invention, or encoding the bispecific antibody according to any embodiment of the present invention. Yet another aspect of the present invention relates to a recombinant vector comprising the isolated nucleic acid molecule of the present invention.

Yet another aspect of the present invention relates to a host cell comprising the isolated nucleic acid molecule of the present invention or the recombinant vector of the present invention.

Yet another aspect of the present invention relates to a method for preparing the antibody or the antigen-binding fragment thereof according to any embodiment of the present invention or the bispecific antibody according to any embodiment of the present invention, comprising: culturing the host cell of the present invention in a suitable condition, and isolating the antibody or the antigen-binding fragment thereof or the bispecific antibody from the cell cultures.

Yet another aspect of the present invention relates to a pharmaceutical composition comprising the antibody or the antigen-binding fragment thereof according to any embodiment of the present invention, the antibody-drug conjugate according to any embodiment of the present invention, or the bispecific antibody according to any embodiment of the present invention, wherein optionally, the pharmaceutical composition further comprises a pharmaceutically acceptable auxiliary material.

Yet another aspect of the present invention relates to use of the antibody or the antigen-binding fragment thereof according to any embodiment of the present invention, the antibody-drug conjugate according to any embodiment of the present invention, or the bispecific antibody according to any embodiment of the present invention in the preparation of a medicament for treating and/or preventing a tumor or anemia, wherein
preferably, the tumor is selected from one or more of ovarian cancer, esophageal cancer, melanoma, a hematological malignancy, glioblastoma, renal cell carcinoma, lung cancer, prostate cancer, bladder cancer, colon cancer, rectal cancer, liver cancer, gastrointestinal cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

The antibody or the antigen-binding fragment thereof according to any embodiment of the present invention, the antibody-drug conjugate according to any embodiment of the present invention, or the bispecific antibody according to any embodiment of the present invention is for use in treating and/or preventing a tumor or anemia, wherein
preferably, the tumor is selected from one or more of ovarian cancer, esophageal cancer, melanoma, a hematological malignancy, glioblastoma, renal cell carcinoma, lung cancer, prostate cancer, bladder cancer, colon cancer, rectal cancer, liver cancer, gastrointestinal cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

Yet another aspect of the present invention relates to a method for treating and/or preventing a tumor or anemia, comprising a step of administering to a subject in need an effective amount of the antibody or the antigen-binding fragment thereof according to any embodiment of the present invention, the antibody-drug conjugate according to any embodiment of the present invention, or the bispecific antibody according to any embodiment of the present invention, wherein
preferably, the tumor is selected from one or more of ovarian cancer, esophageal cancer, melanoma, a hematological malignancy, glioblastoma, renal cell carcinoma, lung cancer, prostate cancer, bladder cancer, colon cancer, rectal cancer, liver cancer, gastrointestinal cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

In the present invention, unless otherwise defined, the scientific and technical terms used herein have the meanings generally understood by those skilled in the art. In addition, the laboratory operations of cell culture, molecular genetics, nucleic acid chemistry and immunology used herein are the routine procedures widely used in the corresponding fields. Meanwhile, in order to better understand the present invention, the definitions and explanations of the relevant terms are provided below.

As used herein, the term EC₅₀ refers to the concentration for 50% of maximal effect, i.e., the concentration that can cause 50% of the maximal effect.

As used herein, the term "antibody" refers to an immunoglobulin molecule that generally consists of two pairs of polypeptide chains (each pair with one "light" (L) chain and one "heavy" (H) chain). Antibody light chains are classified into κ and λ light chains. Heavy chains are classified into µ, δ, γ, α, or ε. Isotypes of antibodies are defined as IgM, IgD, IgG, IgA, and IgE. In light chains and heavy chains, the variable region and constant region are linked by a "J" region of about 12 or more amino acids, and the heavy chain further comprises a "D" region of about 3 or more amino acids. Each heavy chain consists of a heavy chain variable region (VH) and a heavy chain constant region (CH). The heavy chain constant region consists of 3 domains (CH1, CH2, and CH3). Each light chain consists of a light chain variable region (VL) and a light chain constant region (CL). The light chain constant region consists of one domain CL. The constant region of the antibody can mediate the binding of immunoglobulins to host tissues or factors, including the binding of various cells of the immune system (e.g., effector cells) to the first component (C1q) of the classical complement system. The VH and VL regions can be further subdivided into hypervariable regions (called complementarity determining regions (CDRs)), between which conservative regions called framework regions (FRs) are distributed. Each VH and VL consists of 3 CDRs and 4 FRs arranged from amino terminus to carboxyl terminus in the following order: FR1, CDR1, FR2, CDR2, FR3, CDR3, and FR4. The variable regions (VH and VL) of each heavy chain/light chain pair form an antibody-binding site. The assignment of amino acids to the regions or domains is based on Bethesda M.d., Kabat Sequences of Proteins of Immunological Interest (National Institutes of Health, (1987 and 1991)), or Chothia & Lesk J. Mol. Biol, 1987; 196: 901-917; Chothia et al., Nature, 1989; 342: 878-883, or the definition of the IMGT numbering system, see the definition in Ehrenmann F, Kaas Q, Lefranc M P., IMGT/3Dstructure-DB and IMGT/DomainGapAlign: a database and a tool for immunoglobulins or antibodies, T cell receptors, MHC, IgSF and MhcSF[J]., Nucleic acids research, 2009; 38(suppl_1): D301-D307.

In particular, the heavy chain may further comprise more than 3 CDRs, such as 6, 9, or 12. For example, in the bispecific antibody of the present invention, the heavy chain may be a heavy chain of an IgG antibody with the C-terminus linked to one ScFv, and in this case, the heavy chain comprises 9 CDRs.

The term "antibody" is not limited by any specific method for producing the antibody. For example, the antibody includes a recombinant antibody, a monoclonal antibody, and a polyclonal antibody. The antibody may be antibodies of different isotypes, such as IgG (e.g., subtype IgG1, IgG2, IgG3, or IgG4), IgA1, IgA2, IgD, IgE, or IgM.

As used herein, the terms "mAb" and "monoclonal antibody" refer to an antibody or a fragment of an antibody that is derived from a group of highly homologous antibodies, i.e., from a group of identical antibody molecules, except for natural mutations that may occur spontaneously. The monoclonal antibody is highly specific for a single epitope on an antigen. The polyclonal antibody, relative to the monoclonal antibody, generally comprises at least 2 or more different antibodies which generally recognize different epitopes on an antigen. Monoclonal antibodies can generally be obtained using hybridoma technology first reported by Kohler et al. (Köhler G, Milstein C. Continuous cultures of fused cells secreting antibody of predefined specificity [J]. Nature, 1975; 256(5517): 495), but can also be obtained using recombinant DNA technology (see, e.g., U.S. Patent 4,816,567).

As used herein, the term "humanized antibody" refers to an antibody or antibody fragment obtained when all or a part of CDRs of a human immunoglobulin (receptor antibody) is replaced by the CDRs of a non-human antibody (donor antibody), wherein the donor antibody may be a non-human (e.g., mouse, rat or rabbit) antibody having expected specificity, affinity or reactivity. In addition, some amino acid residues in the framework regions (FRs) of the receptor antibody can also be replaced by the amino acid residues of corresponding non-human antibodies or by the amino acid residues of other antibodies to further improve or optimize the performance of the antibody. For more details on humanized antibodies, see, e.g., Jones et al., Nature, 1986; 321: 522-525; Reichmann et al., Nature, 1988; 332: 323-329; Presta, Curr. Op. Struct. Biol., 1992; 2: 593-596; and Clark, Immunol. Today, 2000; 21: 397-402. In some cases, the antigen-binding fragment of the antibody is a diabody, in which the V_{H} and V_{L} domains are expressed on a single polypeptide chain. However, the linker used is too short to allow the pairing of the two domains on the same chain. Thus the domains are forced to pair with the complementary domains on the other chain and two antigen-binding sites are generated (see, e.g., Holliger P. et al., Proc. Natl. Acad. Sci. USA, 1993; 90: 6444-6448 and Poljak R.J. et al., Structure, 1994; 2: 1121-1123).

As used herein, the term "single chain fragment variable (ScFv)" refers to a molecule in which the antibody heavy chain variable region (V_{H}) and the antibody light chain variable region (V_{L}) are linked by a linker. The V_{L} and V_{H} domains are paired to form a monovalent molecule by a linker that enables them to produce a single polypeptide chain (see, e.g., Bird et al., Science, 1988; 242:423-426 and Huston et al., Proc. Natl. Acad. Sci. USA, 1988; 85:5879-5883). Such scFv molecules may have the general structure: NH2-V_{L}-linker fragment-V_{H}-COOH or NH2-V_{H}-linker fragment-V_{L}-COOH. An appropriate linker in the prior art consists of GGGGS amino acid sequence repeats or a variant thereof. For example, a linker having the amino acid sequence (GGGGS)4 may be used, but variants thereof may also be used (Holliger et al., Proc. Natl. Acad. Sci. USA, 1993; 90: 6444-6448). Other linkers that can be used in the present invention are described by Alfthan et al., Protein Eng., 1995; 8: 725-731, Choi et al., Eur. J. Immunol., 2001; 31:94-106, Hu et al., Cancer Res., 1996; 56: 3055-3061, Kipriyanov et al., J. Mol. Biol, 1999; 293: 41-56 and Roovers et al., Cancer Immunology, Immunotherapy, 2001, 50(1): 51-59.

As used herein, the term "isolated" refers to obtaining by artificial means from a natural state. If a certain "isolated" substance or component is present in nature, it may be the case that a change occurs in its natural environment, or that it is isolated from the natural environment, or both. For example, a certain non-isolated polynucleotide or polypeptide naturally occurs in a certain living animal, and the same polynucleotide or polypeptide with high purity isolated from such a natural state is referred to as an isolated polynucleotide or polypeptide. The term "isolated" does not exclude the existence of artificial or synthetic substances or other impurities that do not affect the activity of the substance.

As used herein, the term "vector" refers to a nucleic acid vehicle into which a polynucleotide can be inserted. When a vector allows the expression of the protein encoded by the inserted polynucleotide, the vector is referred to as an expression vector. The vector can be introduced into a host cell by transformation, transduction or transfection, such that the genetic substance elements carried by the vector can be expressed in the host cell. Vectors are well known to those skilled in the art, including but not limited to: plasmids; phagemids; cosmids; artificial chromosomes, such as yeast artificial chromosome (YAC), bacterial artificial chromosome (BAC), or P1-derived artificial chromosome (PAC); phages such as lambda phages or M13 phages; and animal viruses. Animal viruses that can be used as vectors include, but are not limited to retroviruses (including lentiviruses), adenoviruses, adeno-associated viruses, herpes viruses (such as herpes simplex virus), poxviruses, baculoviruses, papillomaviruses, and papovaviruses (such as SV40). A vector may comprise a variety of elements that control expression, including, but not limited to, promoter sequences, transcription initiation sequences, enhancer sequences, selection elements, and reporter genes. In addition, the vector may further comprise a replication initiation site. As used herein, the term "host cell" refers to cells to which vectors can be introduced, including, but not limited to, prokaryotic cells such as *E*. *coli* or *bacillus subtilis,* fungal cells such as yeast cells or *aspergillus,* insect cells such as S2 drosophila cells or Sf9, or animal cells such as fibroblasts, CHO cells, GS cells, COS cells, NSO cells, HeLa cells, BHK cells, HEK 293 cells, or human cells.

As used herein, the term "specific binding" refers to a non-random binding reaction between two molecules, such as a reaction between an antibody and an antigen it targets. In some embodiments, an antibody specifically binding to an antigen (or an antibody specific to an antigen) means that the antibody binds to the antigen with an affinity (K_{D}) of less than about 10⁻⁵ M, such as less than about 10⁻⁶ M, 10⁻⁷ M, 10⁻⁸ M, 10⁻⁹ M, or 10⁻¹⁰ M, or less.

As used herein, the term "K_{D}" refers to a dissociation equilibrium constant for a specific antibody-antigen interaction, which is used to describe the binding affinity between the antibody and the antigen. A smaller dissociation equilibrium constant indicates a stronger antibody-antigen binding and a higher affinity between the antibody and the antigen. Generally, antibodies bind to antigens (e.g., PD-1 protein) with a dissociation equilibrium constant (K_{D}) of less than about 10⁻⁵ M, such as less than about 10⁻⁶ M, 10⁻⁷ M, 10⁻⁸ M, 10⁻⁹ M, or 10⁻¹⁰ M, or less. K_{D} can be determined using methods known to those skilled in the art, e.g., using a Fortebio molecular interaction instrument.

As used herein, the terms "monoclonal antibody" and "mAb" have the same meaning and can be used interchangeably; the terms "polyclonal antibody" and "pAb" have the same meaning and can be used interchangeably. Besides, as used herein, amino acids are generally represented by single-letter and three-letter abbreviations known in the art. For example, alanine can be represented by A or Ala.

As used herein, the term "pharmaceutically acceptable carrier and/or excipient" refers to a carrier and/or excipient that is pharmacologically and/or physiologically compatible with the subject and the active ingredient. Such carriers and/or excipients are well known in the art (see, e.g., Remington's Pharmaceutical Sciences, edited by Gennaro AR, 19th Ed., Pennsylvania, Mack Publishing Company, 1995), including but not limited to: pH regulators, surfactants, adjuvants and ionic strength enhancers. For example, the pH regulators include, but are not limited to, phosphate buffer; the surfactants include, but are not limited to, cationic, anionic or non-ionic surfactants, such as Tween-80; the ionic strength enhancers include, but are not limited to, sodium chloride.

As used herein, the term "effective amount" refers to an amount sufficient to obtain or at least partially obtain a desired effect. For example, a prophylactically effective amount against a disease (e.g., a tumor) refers to an amount sufficient to prevent, stop, or delay the onset of the disease (e.g., a tumor); a therapeutically effective amount refers to an amount sufficient to cure or at least partially stop diseases and complications thereof in patients suffering from the disease. It is undoubtedly within the ability of those skilled in the art to determine such an effective amount. For example, the amount effective for therapeutic purpose will depend on the severity of the disease to be treated, the overall state of the patient's own immune system, the general condition of the patient such as age, body weight and gender, the route of administration, and other treatments given concurrently, etc.

As used herein, when referring to the amino acid sequence of PD-1 protein (NCBI GenBank: NM 005018), it includes the full length of PD-1 protein, or the extracellular fragment PD-1 ECD of PD-1, or a fragment comprising PD-1 ECD, and it also includes a fusion protein of the full length of PD-1 protein or a fusion protein of PD-1 ECD, such as a fragment fused to an Fc protein fragment of mouse or human IgG (mFc or hFc). However, those skilled in the art will appreciate that in the amino acid sequence of the PD-1 protein, mutations or variations (including but not limited to, substitutions, deletions, and/or additions) can be naturally produced or artificially introduced without affecting biological functions thereof. Therefore, in the present invention, the term "PD-1 protein" should include all such sequences, including their natural or artificial variants. In addition, when describing a sequence fragment of the PD-1 protein, it also includes the corresponding sequence fragments in their natural or artificial variants.

As used herein, when referring to the amino acid sequence of lymphocyte-activation gene 3 (LAG3), it includes the full length of LAG3 protein, or the extracellular fragment LAG3 ECD of LAG3, or a fragment comprising LAG3 ECD, and it also includes a fusion protein of the full length of LAG3 protein or a fusion protein of LAG3 ECD, such as a fragment fused to an Fc protein fragment of mouse or human IgG (mFc or hFc). However, those skilled in the art will appreciate that in the amino acid sequence of the LAG3 protein, mutations or variations (including but not limited to, substitutions, deletions, and/or additions) can be naturally produced or artificially introduced without affecting biological functions thereof. Therefore, in the present invention, the term "LAG3 protein" should include all such sequences, including their natural or artificial variants. In addition, when describing a sequence fragment of the LAG3 protein, it also includes the corresponding sequence fragments in their natural or artificial variants.

In the present invention, the terms "first" (e.g., first protein functional region) and "second" (e.g., second protein functional region) are used for distinguishing or clarity in expression and do not carry typical sequential meanings, unless otherwise specified.

### Beneficial effects of the present invention

The present invention achieves one or more of the following effects:
(1) the anti-LAG3 antibody of the present invention has superior affinity and specificity;
(2) the bispecific antibodies of the present invention (e.g., BS-PL021A, BS-PL022B, or BS-PL023C) can specifically bind to LAG3, and can effectively block the binding of LAG3 to MHC II, and specifically relieve the immunosuppression of LAG3 on an organism;
(3) the bispecific antibodies of the present invention (e.g., BS-PL021A, BS-PL022B, or BS-PL023C) can specifically bind to PD-1, and can effectively block the binding of PD-1 to PDL1, and specifically relieve the immunosuppression of PD-1 on an organism and activate immune responses;
(4) the first protein functional region and the second protein functional region in the bispecific antibody of the present invention have a synergistic effect;
(5) the bispecific antibodies of the present invention, particularly BS-PL022B, completely eliminate the binding activity thereof to Fc receptors FcyRI, FcyRIIb, FcyRIIa_H131, FcγRIIIa_V158, and/or FcγRIIIa_F158, and further completely eliminate the ADCC activity or ADCP activity thereof; and
(6) the bispecific antibodies of the present invention, particularly BS-PL022B, completely eliminate the binding activity thereof to a complement C1q, and further eliminate the CDC activity thereof.

### BRIEF DESCRIPTION OF THE DRAWINGS

FIG. 1: Results of assays for the binding activity of BS-PL021A, BS-PL022B, BS-PL023C, Bs-PLV02, and 14C12H1L1(hG1TM) to antigen PD-1-mFc by indirect ELISA.
FIG. 2: Results of assays for the binding activity of BS-PL021A, BS-PL022B, BS-PL023C, BS-PLV02, Relatlimab, and H7L8(hG1WT) to antigen LAG3-mFc by indirect ELISA.
FIG. 3: Results of assays for the activity of anti-LAG3/anti-PD-1 bispecific antibodies in competing with human PDL1-mFc for binding to human PD-1-mFc-Biotin by competitive ELISA.
FIG. 4: Results of assays for the binding activity of anti-LAG3/anti-PD-1 bispecific antibodies to PD-1 on 293T-PD1 membrane surface by FACS.
FIG. 5: Results of an assay for the binding activity of an anti-LAG3/anti-PD-1 bispecific antibody to LAG3 on 293T-LAG3 membrane surface by FACS.
FIG. 6: Results of an assay for the activity of an anti-LAG3/anti-PD-1 bispecific antibody in competing with PDL1 for binding to antigen PD-1 on cell membrane surface by competitive flow cytometry.
FIG. 7: Results of an assay for the activity of an anti-LAG3/anti-PD-1 bispecific antibody in competing with LAG3 for binding to antigen MHC II on cell membrane surface by competitive flow cytometry.
FIG. 8A: Results of assays for anti-LAG3/anti-PD-1 bispecific antibodies blocking the binding of LAG3 to MHCII.
FIG. 8B: Results of assays for anti-LAG3/anti-PD-1 bispecific antibodies blocking the binding of LAG3 to MHCII.
FIG. 9A: Results of an assay for an anti-LAG3/anti-PD-1 bispecific antibody blocking the binding of PD-1 to PD-L1.
FIG. 9B: Results of an assay for an anti-LAG3/anti-PD-1 bispecific antibody blocking the binding of PD-1 to PD-L1.
FIG. 10A: Results of an assay for an anti-LAG3/anti-PD-1 bispecific antibody simultaneously blocking the binding of LAG3 to MHCII and PD-1 to PD-L1.
FIG. 10B: Results of an assay for an anti-LAG3/anti-PD-1 bispecific antibody simultaneously blocking the binding of LAG3 to MHCII and PD-1 to PD-L1.
FIG. 11: Results of a bridging assay for an anti-LAG3/anti-PD-1 bispecific antibody.
FIG. 12A. Results of an assay for the biological activity of an anti-LAG3/anti-PD-1 bispecific antibody in promoting IFN-γ secretion by mixed lymphocyte reaction (MLR).
FIG. 12B. Results of an assay for the biological activity of an anti-LAG3/anti-PD-1 bispecific antibody in promoting IL-2 secretion by mixed lymphocyte reaction (MLR).
FIG. 13: Results of an assay for affinity constants of BS-PL022B to FcyRI.
FIG. 14: Results of an assay for affinity constants of H7L8(hG1WT) to FcyRI.
FIG. 15: Results of an assay for affinity constants of BS-PL022B to FcyRIHa_V158.
FIG. 16: Results of an assay for affinity constants of H7L8(hG1WT) to FcyRHIa_V158.
FIG. 17: Results of an assay for affinity constants of BS-PL022B to FcγRIIIa_F158.
FIG. 18: Results of an assay for affinity constants of H7L8(hG 1 WT) to FcyRHIa_F158.
FIG. 19: Results of an assay for affinity constants of BS-PL022B to FcγRIIa_H131.
FIG. 20: Results of an assay for affinity constants of H7L8(hG1WT) to FcγRIIa_H131.
FIG. 21: Results of an assay for affinity constants of BS-PL022B to FcyRIIb.
FIG. 22: Results of an assay for affinity constants of H7L8(hG1WT) to FcγRIIb.
FIG. 23: Results of an assay for affinity constants of BS-PL022B to C1q.
FIG. 24: Results of an assay for affinity constants of H7L8(hG1WT) to C1q.
FIG. 25: Results of an ADCP effect assay for BS-PL022B.
FIG. 26: Efficacy of anti-LAG3/anti-PD-1 bispecific antibodies in a BALB/c-hPD1/hLAG3 mouse model grafted with CT26 tumor. * P < 0.05, ** P < 0.01, *** P < 0.001, VS isotype control group (two-way ANOVA)
FIG. 27: Effects of anti-LAG3/anti-PD-1 bispecific antibodies on body weight in a BALB/c-hPD1/hLAG3 mouse model grafted with CT26 tumor.

### DETAILED DESCRIPTION

The embodiments of the present invention will be described in detail below with reference to the examples. Those skilled in the art will appreciate that the following examples are only for illustrating the present invention, and should not be construed as limitations to the scope of the present invention. Examples where the specific technologies or conditions are not specified are performed according to the technologies or conditions described in the publications of the art (e.g., see, Molecular Cloning: A Laboratory Manual, authored by J. Sambrook et al., and translated by Huang Peitang et al., third edition, Science Press) or according to the package insert. Reagents or instruments used are commercially available conventional products if the manufacturers thereof are not specified. For example, MDA-MB-231 cells and U87-MG cells could be purchased from ATCC.

BALB/c mice were purchased from Guangdong Medical Laboratory Animal Center. Nivolumab was purchased from BMS, with Batch No. ABA0330. Nivolumab is an anti-PD-1 antibody.

Pembrolizumab was purchased from MSD Ireland (Carlow), with Cat. No. S023942. Pembrolizumab is an anti-PD-1 antibody.

The positive control antibody, Relatlimab, has sequences referenced to the U.S. Patent Publication No. US20160326248A1, wherein the heavy chain amino acid sequence is referenced to SEQ ID NO: 1 of this patent publication and the light chain amino acid sequence is referenced to SEQ ID NO: 2 of this patent publication. Relatlimab is an anti-LAG-3 antibody.

The cell line 293T-PD1 was constructed by Akeso Biopharma Inc. The cell line 293T-PD1 was produced by viral infection of HEK293T cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was plenti6.3/V5-PD1FL-BSD (PD1, Genebank ID: NM 005018; vector plenti6.3/V5-BSD, purchased from Invitrogen, Cat. No. K5315-20).

The cell line 293T-LAG3 was constructed by Akeso Biopharma Inc. The cell line 293T-LAG3 was produced by viral infection of HEK293T cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was plenti6.3/V5-huLAG3FL-BSD (LAG3, Genebank ID: NM 002277.4; vector plenti6.3/V5-BSD, purchased from Invitrogen, Cat. No. K5315-20).

The cell line Raji-PDL1 was constructed by Akeso Biopharma Inc. The cell line Raji-PDL1 was produced by viral infection of Raji cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was plenti6.3/V5-PDL1 (PDL1, Genebank ID: NP_054862.1; vector plenti6.3/V5, purchased from Invitrogen, Cat. No. K5315-20).

The cell line Jurkat-NFAT-PD1-LAG3 was constructed by Akeso Biopharma Inc. The cell line Jurkat-NFAT-PD1-LAG3 was prepared by viral infection of PD-1 effector cells (CPM, manufacturer: Promega, Cat. No. J112A) using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was pCDH-huLAG3FL-RFP-NEO (LAG3, Genebank ID: NM 002277.4; vector pCDH-CMV-MCS-EF1-RFP+Neo, purchased from Youbio, Cat. No. VT9005).

The cell line CHO-K1-PD1 was constructed by Akeso Biopharma Inc. The cell line CHO-K1-PD1 was prepared by viral infection of CHO-K1 cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was pCDH-CMV-PD-1FL-Puro (PD1, Genebank ID: NM 005018; vector pCDH-CMV-Puro, purchased from Youbio, Cat. No. VT1480).

The cell line CHO-K1-LAG3 was constructed by Akeso Biopharma Inc. The cell line CHO-K1-LAG3 was produced by viral infection of CHO-K1 cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vector used was plenti6.3/V5-huLAG3FL-BSD (LAG3, Genebank ID: NM 002277.4; vector plenti6.3/V5-BSD, purchased from Invitrogen, Cat. No. K5315-20).

The cell line Jurkat-NFAT-CD64-CD32R was constructed by Akeso Biopharma Inc. The cell line Jurkat-NFAT-CD64-CD32R was prepared by viral infection of Jurkat cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vectors used were pCDH-NFAT-Hygro (vector pCDH-Hygro, obtained by modifying based on pCDH-CMV-MCS-EF1-Puro (purchased from Youbio, Cat. No. VT1480) in our laboratory), pcDH-hFCGR1AFL-Neo (vector pCDH-Neo, obtained by modifying based on pCDH-CMV-MCS-EF1-Puro (purchased from Youbio, Cat. No. VT1480) in our laboratory), and pCDH-hFCGR2A(H167)-puro (hFCGR2A(H167), Genebank ID: P12318; vector pCDH-CMV-MCS-EF1-Puro, purchased from Youbio, Cat. No. VT1480).

The cell line CHO-K1-PD1-LAG3 was constructed by Akeso Biopharma Inc. The cell line CHO-K1-PD1-LAG3 was prepared by viral infection of CHO-K1 cells using 3rd Generation Lentiviral Systems (see, e.g., A Third Generation Lentivirus Vector with a Conditional Packaging System. Dull T, Zufferey R, Kelly M, Mandel RJ, Nguyen M, Trono D, and Naldini L., J Virol., 1998. 72(11): 8463-8471), wherein the lentivirus expression vectors used were pCDH-hPD1-FL-puro (PD-1, Genebank ID: NM 005018; vector pCDH-CMV-MCS-EF1-Puro, purchased from Youbio, Cat. No. VT1480) and plenti6.3/V5-huLAG3FL-BSD (LAG3, Genebank ID: NM 002277.4; vector plenti6.3/V5-BSD, purchased from Invitrogen, Cat. No. K5315-20).

### Prenaration Examnle 1: Design and Prenaration of Anti-LAG3 Antibodies

### 1. Design of antibodies

The inventors creatively designed a series of antibody sequences based on the known LAG3 protein sequence (NCBI Reference Sequence: NP_002277.4) and the three-dimensional crystal structure thereof, etc. Through extensive screening and testing, humanized monoclonal antibodies specifically binding to LAG3 were finally obtained, named H7L8, H7L9 and H7L10, respectively. The amino acid sequences of the heavy and light chain variable regions of the monoclonal antibodies and the encoding sequences thereof are as follows.
Nucleotide sequence of the heavy chain variable region H7v of H7L8 (360 bp): 
CAGGTGCAGCTGCAGCAGTGGGGAGCTGGACTGCTGAAACCTAGCGAGACACT
GAGCCTGACCTGTGCTGTGTACGGCGGATCTATCAGCGATTACTACTGGAACT
GGATCAGGCAGCCCCCTGGAAAGGGACTGGAATGGATCGGAGAGATCAACCAC
AGGGGCACCACCAACTCCAATCCCTCTCTGAAGAGCAGGGTGACACTGAGCCT
CGACACAAGCAAGAATCAGTTCAGCCTGAAGCTGAGGTCCGTGACCGCTGCTG
 
ATACAGCTGTGTACTACTGTGCCTTCGGCTACAGCGATTACGAGTACGATTGGT
TCGACCCTTGGGGCCAGGGAACACTGGTTACAGTGAGCTCC (SEQ ID NO: 1)
Amino acid sequence of the heavy chain variable region H7v of H7L8 (120 aa): 
QVQLQQWGAGLLKPSETLSLTCAVYGGSISDYYWNWIRQPPGKGLEWIGEINHRG
TTNSNPSLKSRVTLSLDTSKNQFSLKLRSVTAADTAVYYCAFGYSDYEYDWFDPWG
QGTLVTVSS (SEQ ID NO: 2)
Nucleotide sequence of the light chain variable region L8v of H7L8 (321 bp): 
GAGATCGTTCTGACCCAGAGCCCAGCTACACTGAGCCTGTCTCCTGGAGAGAG
GGCTACACTGTCCTGCAGAGCTAGCCAGACCATCAGCAGCTACCTGGCTTGGT
ACCAGCAGAAGCCTGGCCAAGCTCCAAGGCTGCTGATCTACGACGCCTCTAAT
AGGGCCACCGGCATCCCTGCTAGATTCTCTGGAAGCGGCAGCGGAACCGACTT
TACACTGACAATCAGCTCCCTGGAGCCCGAGGATTTCGCTGTTTACTACTGTCA
GCAGCGCAGCAACTGGCCCATCACATTCGGACAGGGCACAAATCTGGAGATCA
AG (SEQ ID NO: 3)
Amino acid sequence of the light chain variable region L8v of H7L8 (107 aa):

EIVLTQSPATLSLSPGERATLSCRASQTISSYLAWYQQKPGQAPRLLIYDASNRATGI
PARFSGSGSGTDFTLTISSLEPEDFAVYYCQQRSNWPITFGQGTNLEIK (SEQID NO:
4)

The nucleotide sequence of the heavy chain variable region H7v of H7L9 is identical to the nucleotide sequence of the heavy chain variable region H7v of H7L8, as set forth in SEQ ID NO: 1.

The amino acid sequence of the heavy chain variable region H7v of H7L9 is identical to the amino acid sequence of the heavy chain variable region H7v of H7L8, as set forth in SEQ ID NO: 2.
Nucleotide sequence of the light chain variable region L9v of H7L9 (321 bp): 
GAGATCGTTCTGACCCAGAGCCCAGCTACACTGAGCCTGTCTCCTGGAGAGAG
GGCTACACTGTCCTGCAGAGCTAGCCAGACCATCAGCAGCTACCTGGCTTGGT
ACCAGCAGAAGCCTGGCCAAGCTCCAAGGCTGCTGATCTACGACGGCTCTAAT
 
AGGGCCACCGGCATCCCTGCTAGATTCTCTGGAAGCGGCAGCGGAACCGACTT
TACACTGACAATCAGCTCCCTGGAGCCCGAGGATTTCGCTGTTTACTACTGTCA
GCAGCGCAGCAACTGGCCCCTCACATTCGGACAGGGCACAAATCTGGAGATCA
AG (SEQ ID NO: 41)
Amino acid sequence of the light chain variable region L9v of H7L9 (107 bp):

EIVLTQSPATLSLSPGERATLSCRASQTISSYLAWYQQKPGQAPRLLIYDGSNRATGI
PARFSGSGSGTDFTLTISSLEPEDFAVYYCQQRSNWPLTFGQGTNLEIK (SEQ ID NO:
42)

The nucleotide sequence of the heavy chain variable region H7v of H7L10 is identical to the nucleotide sequence of the heavy chain variable region H7v of H7L8, as set forth in SEQ ID NO: 1.

The amino acid sequence of the heavy chain variable region H7v of H7L10 is identical to the amino acid sequence of the heavy chain variable region H7v of H7L8, as set forth in SEQ ID NO: 2.
Nucleotide sequence of the light chain variable region L10v of H7L10 (321 bp): 
GAGATCGTTCTGACCCAGAGCCCAGCTACACTGAGCCTGTCTCCTGGAGAGAG
GGCTACACTGTCCTGCAGAGCTAGCCAGTCCATCAGCAGCTACCTGGCTTGGT
ACCAGCAGAAGCCTGGCCAAGCTCCAAGGCTGCTGATCTACGACGGCTCTAAT
AGGGCCACCGGCATCCCTGCTAGATTCTCTGGAAGCGGCAGCGGAACCGACTT
TACACTGACAATCAGCTCCCTGGAGCCCGAGGATTTCGCTGTTTACTACTGTCA
GCAGCGCAGCAACTGGCCCATCACATTCGGACAGGGCACAAATCTGGAGATCA
AG (SEQ ID NO: 43)
Amino acid sequence of the light chain variable region L10v of H7L10 (107 bp):

EIVLTQSPATLSLSPGERATLSCRASQSISSYLAWYQQKPGQAPRLLIYDGSNRATGI
PARFSGSGSGTDFTLTISSLEPEDFAVYYCQQRSNWPITFGQGTNLEIK (SEQ ID NO:
44)

The amino acid sequences of the CDRs of the antibody H7L8 are as follows (according to the IMGT numbering system):
HCDR1: GGSISDYY (SEQ ID NO: 5);
HCDR2: INHRGTT (SEQ ID NO: 6);
HCDR3: AFGYSDYEYDWFDP (SEQ ID NO: 7);
LCDR1: QTISSY (SEQ ID NO: 8);
LCDR2: DAS (SEQ ID NO: 9); and
LCDR3: QQRSNWPIT (SEQ ID NO: 10).

The amino acid sequences of the CDRs of the antibody H7L9 are as follows (according to the IMGT numbering system):
HCDR1: GGSISDYY (SEQ ID NO: 5);
HCDR2: INHRGTT (SEQ ID NO: 6);
HCDR3: AFGYSDYEYDWFDP (SEQ ID NO: 7);
LCDR1: QTISSY (SEQ ID NO: 8);
LCDR2: DGS (SEQ ID NO: 46); and
LCDR3: QQRSNWPLT (SEQ ID NO: 47).

The amino acid sequences of the CDRs of the antibody H7L10 are as follows (according to the IMGT numbering system):
HCDR1: GGSISDYY (SEQ ID NO: 5);
HCDR2: INHRGTT (SEQ ID NO: 6);
HCDR3: AFGYSDYEYDWFDP (SEQ ID NO: 7);
LCDR1: QSISSY (SEQ ID NO: 48);
LCDR2: DGS (SEQ ID NO: 46); and
LCDR3: QQRSNWPIT (SEQ ID NO: 10).

### 2. Expression and purification of humanized antibody H7L8(hG 1 WT)

The heavy chain cDNA sequence (the encoding sequence of the variable region was set forth in SEQ ID NO: 1; the constant region was Ig gamma-1 chain C region, SEQ ID NO: 39) and the light chain cDNA sequence (the encoding sequence of the variable region was set forth in SEQ ID NO: 3; the constant region was P01834.1 (human Ig kappa chain C region, SEQ ID NO: 40) of H7L8(hG1WT) were separately cloned into pUC57simple vectors (supplied by GenScript), and plasmids pUC57simple-H7 and pUC57simple-L8 were obtained, respectively. The plasmids pUC57simple-H7 and pUC57simple-L8 were each digested (HindIII&EcoRI). The heavy and light chains isolated by electrophoresis were separately subcloned into pcDNA3.1 vectors, and recombinant plasmids were extracted to co-transfect 293F cells. After 7 days of cell culture, the culture medium was separated by high-speed centrifugation, and the supernatant was concentrated and loaded onto a HiTrap MabSelect SuRe column. The protein was eluted in one step with an elution buffer. The target sample was isolated, and the buffer was exchanged into PBS.
Amino acid sequence of the heavy chain constant region of H7L8(hG1WT) 





 (SEQ ID
NO: 39)
Amino acid sequence of the light chain constant region of H7L8(hG1WT)


 (SEQ ID
NO: 40)

### 3. Design of humanized antibody H7L8(hG1TM)

On the basis of H7L8(hG1WT), the inventors obtained a humanized antibody H7L8(hG1TM) with constant region mutations by introducing a leucine-to-alanine point mutation at position 234 (according to the EU numbering system, the same below) (L234A), a leucine-to-alanine point mutation at position 235 (L235A), and a glycine-to-alanine point mutation at position 237 (G237A) in the heavy chain. The amino acid sequence of the heavy chain H7(hG1TM) of H7L8(hG1TM) is set forth in SEQ ID NO: 11, and the amino acid sequence of the light chain L8 thereof is set forth in SEQ ID NO: 12.

The humanized antibody H7L8(hG1TM) was prepared by the method described above in step 2.
Amino acid sequence of the heavy chain H7(hG1TM) of H7L8(hG1TM) 
QVQLQQWGAGLLKPSETLSLTCAVYGGSISDYYWNWIRQPPGKGLEWIGEINHRG
TTNSNPSLKSRVTLSLDTSKNQFSLKLRSVTAADTAVYYCAFGYSDYEYDWFDPWG
QGTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDK
THTCPPCPAPEAAGAPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSP
GK (SEQ ID NO: 11)
Amino acid sequence of the light chain L8 of H7L8(hG1TM)

EIVLTQSPATLSLSPGERATLSCRASQTISSYLAWYQQKPGQAPRLLIYDASNRATGI
PARFSGSGSGTDFTLTISSLEPEDFAVYYCQQRSNWPITFGQGTNLEIKRTVAAPSVF
IFPPSDEQLKSGTASVVCLLNNFYPREAKVQWKVDNALQSGNSQESVTEQDSKDST
YSLSSTLTLSKADYEKHKVYACEVTHQGLSSPVTKSFNRGEC (SEQ ID NO: 12)

### 4. Design of humanized antibody H7L8(hG4DM)

On the basis of H7L8(hG1WT), the inventors obtained a humanized antibody H7L8(hG4DM) with constant region mutations by using Ig gamma-4 chain C region as a heavy chain constant region and introducing a phenylalanine-to-alanine point mutation at position 234 (F234A) and a leucine-to-alanine point mutation at position 235 (L235A) in the heavy chain constant region while keeping the antibody variable region unchanged. The amino acid sequence of the heavy chain of H7L8(hG4DM) is set forth in SEQ ID NO: 13, and the amino acid sequence of the light chain thereof is set forth in SEQ ID NO: 12. Amino acid sequence of the heavy chain H7(hG4DM) of H7L8(hG4DM):

QVQLQQWGAGLLKPSETLSLTCAVYGGSISDYYWNWIRQPPGKGLEWIGEINHRG
TTNSNPSLKSRVTLSLDTSKNQFSLKLRSVTAADTAVYYCAFGYSDYEYDWFDPWG
QGTLVTVSSASTKGPSVFPLAPCSRSTSESTAALGCLVKDYFPEPVTVSWNSGALTS
GVHTFPAVLQSSGLYSLSSVVTVPSSSLGTKTYTCNVDHKPSNTKVDKRVESKYGPP
CPPCPAPEAAGGPSVFLFPPKPKDTLMISRTPEVTCVVVDVSQEDPEVQFNWYVDG
VEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLGK
(SEQ ID NO: 13)

The amino acid sequence of the light chain L8 of H7L8(hG4DM) is identical to the amino acid sequence of the light chain of H7L8(hG1TM), as set forth in SEQ ID NO: 12.

### 5. Expression and purification of humanized antibodies H7L8(hG4WT), H7L9(hG4WT) and H7L10(hG4WT)

The heavy chain cDNA sequences (the encoding sequences of the variable regions were set forth in SEQ ID NO: 1; the constant regions were Ig gamma-4 chain C regions, set forth in SEQ ID NO: 45) of H7L8(hG4WT), H7L9(hG4WT) and H7L10(hG4WT), the light chain cDNA sequence (the encoding sequence of the variable region was set forth in SEQ ID NO: 3; the constant region was human Ig kappa chain C region, set forth in SEQ ID NO: 40) of H7L8(hG4WT), the light chain cDNA sequence (the encoding sequence of the variable region was set forth in SEQ ID NO: 42; the constant region was human Ig kappa chain C region, set forth in SEQ ID NO: 40) of H7L9(hG4WT), and the light chain cDNA sequence (the encoding sequence of the variable region was set forth in SEQ ID NO: 44; the constant region was human Ig kappa chain C region, set forth in SEQ ID NO: 40) of H7L10(hG4WT) were separately cloned into pUC57simple vectors (supplied by GenScript), and plasmids pUC57simple-H7, pUC57simple-L8, pUC57simple-L9 and pUC57simple-L10 were obtained, respectively. The plasmids pUC57simple-H7, pUC57simple-L8, pUC57simple-L9 and pUC57simple-L10 were each digested (HindIII&EcoRI). The heavy and light chains isolated by electrophoresis were separately subcloned into pcDNA3.1 vectors, and recombinant plasmids were extracted to co-transfect 293F cells. After 7 days of cell culture, the culture medium was separated by high-speed centrifugation, and the supernatant was concentrated and loaded onto a HiTrap MabSelect SuRe column. The protein was eluted in one step with an elution buffer. The target sample was isolated, and the buffer was exchanged into PBS.
Amino acid sequence of the heavy chain constant region of H7L8(hG4WT), H7L9(hG4WT), or H7L10(hG4WT): 
ASTKGPSVFPLAPCSRSTSESTAALGCLVKDYFPEPVTVSWNSGALTSGVHTFPAVL
QSSGLYSLSSVVTVPSSSLGTKTYTCNVDHKPSNTKVDKRVESKYGPPCPPCPAPEF
LGGPSVFLFPPKPKDTLMISRTPEVTCVVVDVSQEDPEVQFNWYVDGVEVHNAKT
KPREEQFNSTYRVVSVLTVLHQDWLNGKEYKCKVSNKGLPSSIEKTISKAKGQPRE
PQVYTLPPSQEEMTKNQVSLTCLVKGFYPSDIAVEWESNGQPENNYKTTPPVLDSD
GSFFLYSRLTVDKSRWQEGNVFSCSVMHEALHNHYTQKSLSLSLGK (SEQ ID NO:
45)
Amino acid sequence of the light chain constant region of H7L8(hG4WT), H7L9(hG4WT), or H7L10(hG4WT):


 (SEQ ID
NO: 40)

### Prenaration Examnle 2: Design and Prenaration of Anti-PD-1 Antibody 14C12 and Its Humanized Antibody 14C12H1L1

The amino acid sequences and encoding nucleotide sequences of the heavy and light chains of the anti-PD-1 antibody 14C12 and its humanized antibody 14C12H1L1 are identical to those of 14C12 and 14C12H1L1 in Chinese Patent Publication No. CN 106967172A (or No. CN 106977602A), respectively.

### (1) Heavy and light chain variable region sequences of 14C12

Nucleotide sequence of the heavy chain variable region of 14C12: (354 bp) 
GAGGTCAAACTGGTGGAGAGCGGCGGCGGGCTGGTGAAGCCCGGCGGGTCAC
TGAAACTGAGCTGCGCCGCTTCCGGCTTCGCCTTTAGCTCCTACGACATGTCAT
GGGTGAGGCAGACCCCTGAGAAGCGCCTGGAATGGGTCGCTACTATCAGCGGA
GGCGGGCGATACACCTACTATCCTGACTCTGTCAAAGGGAGATTCACAATTAG
TCGGGATAACGCCAGAAATACTCTGTATCTGCAGATGTCTAGTCTGCGGTCCG
AGGATACAGCTCTGTACTATTGTGCAAACCGGTACGGCGAAGCATGGTTTGCC
TATTGGGGACAGGGCACCCTGGTGACAGTCTCTGCC (SEQ ID NO: 14)
Amino acid sequence of the heavy chain variable region of 14C12: (118 aa) 
EVKLVESGGGLVKPGGSLKLSCAASGFAFSSYDMSWVRQTPEKRLEWVATISGGG
RYTYYPDSVKGRFTISRDNARNTLYLQMSSLRSEDTALYYCANRYGEAWFAYWGQ
GTLVTVSA (SEQ ID NO: 15)
Nucleotide sequence of the light chain variable region of 14C12: (321 bp) 
GACATTAAGATGACACAGTCCCCTTCCTCAATGTACGCTAGCCTGGGCGAGCG
AGTGACCTTCACATGCAAAGCATCCCAGGACATCAACACATACCTGTCTTGGTT
TCAGCAGAAGCCAGGCAAAAGCCCCAAGACCCTGATCTACCGGGCCAATAGAC
TGGTGGACGGGGTCCCCAGCAGATTCTCCGGATCTGGCAGTGGGCAGGATTAC
TCCCTGACCATCAGCTCCCTGGAGTATGAAGACATGGGCATCTACTATTGCCTG
CAGTATGATGAGTTCCCTCTGACCTTTGGAGCAGGCACAAAACTGGAACTGAA
G (SEQ ID NO: 16)
Amino acid sequence of the light chain variable region of 14C12: (107 aa)

DIKMTQSPSSMYASLGERVTFTCKASQDINTYLSWFQQKPGKSPKTLIYRANRLVD
GVPSRFSGSGSGQDYSLTISSLEYEDMGIYYCLQYDEFPLTFGAGTKLELK (SEQ ID
NO: 17)

### (2) Heavy and light chain variable region sequences and heavy and light chain sequences of humanized monoclonal antibody 14C12H1L1

Nucleotide sequence of the heavy chain variable region 14C12H1v of 14C12H1L1: (354 bp) 
GAAGTGCAGCTGGTCGAGTCTGGGGGAGGGCTGGTGCAGCCCGGCGGGTCAC
TGCGACTGAGCTGCGCAGCTTCCGGATTCGCCTTTAGCTCCTACGACATGTCCT
GGGTGCGACAGGCACCAGGAAAGGGACTGGATTGGGTCGCTACTATCTCAGGA
GGCGGGAGATACACCTACTATCCTGACAGCGTCAAGGGCCGGTTCACAATCTC
TAGAGATAACAGTAAGAACAATCTGTATCTGCAGATGAACAGCCTGAGGGCTG
AGGACACCGCACTGTACTATTGTGCCAACCGCTACGGGGAAGCATGGTTTGCC
TATTGGGGGCAGGGAACCCTGGTGACAGTCTCTAGT (SEQ ID NO: 18)
Amino acid sequence of the heavy chain variable region 14C12H1v of 14C12H1L1: (118 aa) 
EVQLVESGGGLVQPGGSLRLSCAASGFAFSSYDMSWVRQAPGKGLDWVATISGGG
RYTYYPDSVKGRFTISRDNSKNNLYLQMNSLRAEDTALYYCANRYGEAWFAYWG
QGTLVTVSS (SEQ ID NO: 19)
Nucleotide sequence of the light chain variable region 14C12L1v of 14C12H1L1: (321 bp) 
GACATTCAGATGACTCAGAGCCCCTCCTCCATGTCCGCCTCTGTGGGCGACAG
GGTCACCTTCACATGCCGCGCTAGTCAGGATATCAACACCTACCTGAGCTGGTT
TCAGCAGAAGCCAGGGAAAAGCCCCAAGACACTGATCTACCGGGCTAATAGAC
TGGTGTCTGGAGTCCCAAGTCGGTTCAGTGGCTCAGGGAGCGGACAGGACTAC
ACTCTGACCATCAGCTCCCTGCAGCCTGAGGACATGGCAACCTACTATTGCCTG
CAGTATGATGAGTTCCCACTGACCTTTGGCGCCGGGACAAAACTGGAGCTGAA
G (SEQ ID NO: 20)
Amino acid sequence of the light chain variable region 14C12L1v of 14C12H1L1: (107 aa) 
DIQMTQSPSSMSASVGDRVTFTCRASQDINTYLSWFQQKPGKSPKTLIYRANRLVS
GVPSRFSGSGSGQDYTLTISSLQPEDMATYYCLQYDEFPLTFGAGTKLELK (SEQ ID
NO: 21)
Nucleotide sequence of the heavy chain 14C12H1 of 14C12H1L1: (1344 bp) GAAGTGCAGCTGGTCGAGTCTGGGGGAGGGCTGGTGCAGCCCGGCGGGTCAC 
TGCGACTGAGCTGCGCAGCTTCCGGATTCGCCTTTAGCTCCTACGACATGTCCT
GGGTGCGACAGGCACCAGGAAAGGGACTGGATTGGGTCGCTACTATCTCAGGA
GGCGGGAGATACACCTACTATCCTGACAGCGTCAAGGGCCGGTTCACAATCTC
TAGAGATAACAGTAAGAACAATCTGTATCTGCAGATGAACAGCCTGAGGGCTG
AGGACACCGCACTGTACTATTGTGCCAACCGCTACGGGGAAGCATGGTTTGCC
TATTGGGGGCAGGGAACCCTGGTGACAGTCTCTAGTGCCAGCACCAAAGGACC
TAGCGTGTTTCCTCTCGCCCCCTCCTCCAAAAGCACCAGCGGAGGAACCGCTG
CTCTCGGATGTCTGGTGAAGGACTACTTCCCTGAACCCGTCACCGTGAGCTGG
AATAGCGGCGCTCTGACAAGCGGAGTCCATACATTCCCTGCTGTGCTGCAAAG
CAGCGGACTCTATTCCCTGTCCAGCGTCGTCACAGTGCCCAGCAGCAGCCTGG
GCACCCAGACCTACATCTGTAACGTCAACCACAAGCCCTCCAACACCAAGGTG
GACAAGAAAGTGGAGCCCAAATCCTGCGACAAGACACACACCTGTCCCCCCTG
TCCTGCTCCCGAACTCCTCGGAGGCCCTAGCGTCTTCCTCTTTCCTCCCAAACC
CAAGGACACCCTCATGATCAGCAGAACCCCTGAAGTCACCTGTGTCGTCGTGG
ATGTCAGCCATGAGGACCCCGAGGTGAAATTCAACTGGTATGTCGATGGCGTC
GAGGTGCACAACGCCAAAACCAAGCCCAGGGAGGAACAGTACAACTCCACCTA
CAGGGTGGTGTCCGTGCTGACAGTCCTCCACCAGGACTGGCTGAACGGCAAGG
AGTACAAGTGCAAGGTGTCCAACAAGGCTCTCCCTGCCCCCATTGAGAAGACC
ATCAGCAAGGCCAAAGGCCAACCCAGGGAGCCCCAGGTCTATACACTGCCTCC
CTCCAGGGACGAACTCACCAAGAACCAGGTGTCCCTGACCTGCCTGGTCAAGG
GCTTTTATCCCAGCGACATCGCCGTCGAGTGGGAGTCCAACGGACAGCCCGAG
AATAACTACAAGACCACCCCTCCTGTCCTCGACTCCGACGGCTCCTTCTTCCTG
TACAGCAAGCTGACCGTGGACAAAAGCAGGTGGCAGCAGGGAAACGTGTTCTC
CTGCAGCGTGATGCACGAAGCCCTCCACAACCACTACACCCAGAAAAGCCTGT
CCCTGAGCCCCGGCAAA (SEQ ID NO: 22)
Amino acid sequence of the heavy chain 14C12H1 of 14C12H1L1: (448 aa) 
EVQLVESGGGLVQPGGSLRLSCAASGFAFSSYDMSWVRQAPGKGLDWVATISGGG
RYTYYPDSVKGRFTISRDNSKNNLYLQMNSLRAEDTALYYCANRYGEAWFAYWG
QGTLVTVSSASTKGPSVFPLAPSSKSTSGGTAALGCLVKDYFPEPVTVSWNSGALTS
 
GVHTFPAVLQSSGLYSLSSVVTVPSSSLGTQTYICNVNHKPSNTKVDKKVEPKSCDK
THTCPPCPAPELLGGPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSP
GK (SEQ ID NO: 23)
Nucleotide sequence of the light chain 14C12L1 of 14C12H1L1: (642 bp) 
GACATTCAGATGACTCAGAGCCCCTCCTCCATGTCCGCCTCTGTGGGCGACAG
GGTCACCTTCACATGCCGCGCTAGTCAGGATATCAACACCTACCTGAGCTGGTT
TCAGCAGAAGCCAGGGAAAAGCCCCAAGACACTGATCTACCGGGCTAATAGAC
TGGTGTCTGGAGTCCCAAGTCGGTTCAGTGGCTCAGGGAGCGGACAGGACTAC
ACTCTGACCATCAGCTCCCTGCAGCCTGAGGACATGGCAACCTACTATTGCCTG
CAGTATGATGAGTTCCCACTGACCTTTGGCGCCGGGACAAAACTGGAGCTGAA
GCGAACTGTGGCCGCTCCCTCCGTCTTCATTTTTCCCCCTTCTGACGAACAGCT
GAAATCAGGCACAGCCAGCGTGGTCTGTCTGCTGAACAATTTCTACCCTAGAG
AGGCAAAAGTGCAGTGGAAGGTCGATAACGCCCTGCAGTCCGGCAACAGCCAG
GAGAGTGTGACTGAACAGGACTCAAAAGATAGCACCTATTCCCTGTCTAGTAC
ACTGACTCTGTCCAAGGCTGATTACGAGAAGCACAAAGTGTATGCATGCGAAG
TGACACATCAGGGACTGTCAAGCCCCGTGACTAAGTCTTTTAACCGGGGCGAA
TGT (SEQ ID NO: 24)
Amino acid sequence of the light chain 14C12L1 of 14C12H1L1: (214 aa)

DIQMTQSPSSMSASVGDRVTFTCRASQDINTYLSWFQQKPGKSPKTLIYRANRLVS
GVPSRFSGSGSGQDYTLTISSLQPEDMATYYCLQYDEFPLTFGAGTKLELKRTVAA
PSVFIFPPSDEQLKSGTASVVCLLNNFYPREAKVQWKVDNALQSGNSQESVTEQDS
KDSTYSLSSTLTLSKADYEKHKVYACEVTHQGLSSPVTKSFNRGEC (SEQ ID NO: 25)

The CDRs of the antibodies 14C12 and 14C12H1L1 are identical as follows (according to the IMGT numbering system):
HCDR1: GFAFSSYD (SEQ ID NO: 26)
HCDR2: ISGGGRYT (SEQ ID NO: 27)
HCDR3: ANRYGEAWFAY (SEQ ID NO: 28)
LCDR1: QDINTY (SEQ ID NO: 29)
LCDR2: RAN (SEQ ID NO: 30)
LCDR3: LQYDEFPLT (SEQ ID NO: 31)

### Heavy and light chain variable region sequences of 14C12H1L1(M)

14C12H1L1(M) was obtained by mutating certain amino acids in the framework region (light chain) on the basis of 14C12H1L1.

The heavy chain variable region 14C12H1(M) of 14C12H1L1(M) is identical to the heavy chain variable region 14C12H1 of 14C12H1L1, that is, both have an amino acid sequence set forth in SEQ ID NO: 19.

### Light chain variable region 14C12L1(M) of 14C12H1L1(M):

DIQMTQSPSSMSASVGDRVTFTCRASQDINTYLSWFQQKPGKSPKTLIYRANRLVS
GVPSRFSGSGSGQDYTLTISSLQPEDMATYYCLQYDEFPLTFGAGTKLELKR (SEQ
ID NO: 38)

### Preparation Example 3: Design of Humanized Antibody 14C12H1L1(hG4DM)

On the basis of 14C12H1L1, the inventors obtained a humanized antibody 14C12H1L1(hG4DM) with constant region mutations by using Ig gamma-4 chain C region as a heavy chain constant region and introducing a phenylalanine-to-alanine point mutation at position 234 (F234A) and a leucine-to-alanine point mutation at position 235 (L235A) in the heavy chain constant region while keeping the antibody variable region unchanged. The amino acid sequence of the heavy chain 14C12H1(hG4DM) of 14C12H1L1(hG4DM) is set forth in SEQ ID NO: 32, and the amino acid sequence of the light chain thereof is set forth in SEQ ID NO: 25.

### Amino acid sequence of the heavy chain of 14C12H1L1(hG4DM)

EVQLVESGGGLVQPGGSLRLSCAASGFAFSSYDMSWVRQAPGKGLDWVATISGGG

RYTYYPDSVKGRFTISRDNSKNNLYLQMNSLRAEDTALYYCANRYGEAWFAYWG
QGTLVTVSSASTKGPSVFPLAPCSRSTSESTAALGCLVKDYFPEPVTVSWNSGALTS
GVHTFPAVLQSSGLYSLSSVVTVPSSSLGTKTYTCNVDHKPSNTKVDKRVESKYGPP
CPPCPAPEAAGGPSVFLFPPKPKDTLMISRTPEVTCVVVDVSQEDPEVQFNWYVDG
VEVHNAKTKPREEQFNSTYRVVSVLTVLHQDWLNGKEYKCKVSNKGLPSSIEKTIS
KAKGQPREPQVYTLPPSQEEMTKNQVSLTCLVKGFYPSDIAVEWESNGQPENNYK
TTPPVLDSDGSFFLYSRLTVDKSRWQEGNVFSCSVMHEALHNHYTQKSLSLSLGK
(SEQ ID NO: 32)

The amino acid sequence of the light chain of 14C12H1L1(hG4DM) is identical to the amino acid sequence of the light chain 14C12L1 of 14C12H1L1, as set forth in SEQ ID NO: 25.

### Preparation Example 4: Sequence Design of Humanized Antibody 14C12H1L1(hG1TM)

On the basis of the humanized antibody 14C12H1L1, the inventors obtained a humanized mutant 14C12H1L1(hG1TM) by introducing a leucine-to-alanine point mutation at position 234 (L234A), a leucine-to-alanine point mutation at position 235 (L235A), and a glycine-to-alanine point mutation at position 237 (G237A) in the hinge region of the heavy chain according to the EU numbering system.
Nucleotide sequence of the heavy chain 14C12H1(hG1TM) of 14C12H1L1(hG1TM): (1344 bp) 
GAAGTGCAGCTGGTCGAGTCTGGGGGAGGGCTGGTGCAGCCCGGCGGGTCAC
TGCGACTGAGCTGCGCAGCTTCCGGATTCGCCTTTAGCTCCTACGACATGTCCT
GGGTGCGACAGGCACCAGGAAAGGGACTGGATTGGGTCGCTACTATCTCAGGA
GGCGGGAGATACACCTACTATCCTGACAGCGTCAAGGGCCGGTTCACAATCTC
TAGAGATAACAGTAAGAACAATCTGTATCTGCAGATGAACAGCCTGAGGGCTG
AGGACACCGCACTGTACTATTGTGCCAACCGCTACGGGGAAGCATGGTTTGCC
TATTGGGGGCAGGGAACCCTGGTGACAGTCTCTAGTGCCAGCACCAAAGGGCC
CAGCGTGTTTCCTCTCGCCCCCTCCTCCAAAAGCACCAGCGGAGGAACCGCTG
CTCTCGGATGTCTGGTGAAGGACTACTTCCCTGAACCCGTCACCGTGAGCTGG
AATAGCGGCGCTCTGACAAGCGGAGTCCATACATTCCCTGCTGTGCTGCAAAG
 
CAGCGGACTCTATTCCCTGTCCAGCGTCGTCACAGTGCCCAGCAGCAGCCTGG
GCACCCAGACCTACATCTGTAACGTCAACCACAAGCCCTCCAACACCAAGGTG
GACAAGAAAGTGGAGCCCAAATCCTGCGACAAGACACACACCTGTCCCCCCTG
TCCTGCTCCCGAAGCTGCTGGAGCCCCTAGCGTCTTCCTCTTTCCTCCCAAACC
CAAGGACACCCTCATGATCAGCAGAACCCCTGAAGTCACCTGTGTCGTCGTGG
ATGTCAGCCATGAGGACCCCGAGGTGAAATTCAACTGGTATGTCGATGGCGTC
GAGGTGCACAACGCCAAAACCAAGCCCAGGGAGGAACAGTACAACTCCACCTA
CAGGGTGGTGTCCGTGCTGACAGTCCTCCACCAGGACTGGCTGAACGGCAAGG
AGTACAAGTGCAAGGTGTCCAACAAGGCTCTCCCTGCCCCCATTGAGAAGACC
ATCAGCAAGGCCAAAGGCCAACCCAGGGAGCCCCAGGTCTATACACTGCCTCC
CTCCAGGGACGAACTCACCAAGAACCAGGTGTCCCTGACCTGCCTGGTCAAGG
GCTTTTATCCCAGCGACATCGCCGTCGAGTGGGAGTCCAACGGACAGCCCGAG
AATAACTACAAGACCACCCCTCCTGTCCTCGACTCCGACGGCTCCTTCTTCCTG
TACAGCAAGCTGACCGTGGACAAAAGCAGGTGGCAGCAGGGAAACGTGTTCTC
CTGCAGCGTGATGCACGAAGCCCTCCACAACCACTACACCCAGAAAAGCCTGT
CCCTGAGCCCCGGCAAA (SEQ ID NO: 33)
Amino acid sequence of the heavy chain 14C12H1(hG1TM) of 14C12H1L1(hG1TM): (448 aa)

EVQLVESGGGLVQPGGSLRLSCAASGFAFSSYDMSWVRQAPGKGLDWVATISGGG
RYTYYPDSVKGRFTISRDNSKNNLYLQMNSLRAEDTALYYCANRYGEAWFAYWG
QGTLVTVSSASTKGPSVFPLAPSSKSTSGGTAALGCLVKDYFPEPVTVSWNSGALTS
GVHTFPAVLQSSGLYSLSSVVTVPSSSLGTQTYICNVNHKPSNTKVDKKVEPKSCDK
THTCPPCPAPEAAGAPSVFLFPPKPKDTLMISRTPEVTCVVVDVSHEDPEVKFNWY
VDGVEVHNAKTKPREEQYNSTYRVVSVLTVLHQDWLNGKEYKCKVSNKALPAPIE
KTISKAKGQPREPQVYTLPPSRDELTKNQVSLTCLVKGFYPSDIAVEWESNGQPEN
NYKTTPPVLDSDGSFFLYSKLTVDKSRWQQGNVFSCSVMHEALHNHYTQKSLSLSP
GK (SEQ ID NO: 34)

The nucleotide sequence of the light chain of 14C12H1L1(hG1TM) is set forth in SEQ ID NO: 24.

The amino acid sequence of the light chain of 14C12H1L1(hG1TM) is identical to the amino acid sequence of the light chain 14C12L1 of 14C12H1L1, as set forth in SEQ ID NO: 25.

### Prenaration Examnle 5: Design and Prenaration of Anti-LAG3/PD-1 Bifunctional

### Antibodies

### 1. Sequence design

The structures of the bifunctional antibodies BS-PL021A, Bs-PL022B, BS-PL023C and Bs-PLV02 of the present invention are in the Morrison form (IgG-scFv), i.e., C-termini of two heavy chains of an IgG antibody are each linked to an scFv fragment of another antibody, and the main composition design of the heavy and light chains is as shown in Table 1 below.

**Table 1: Composition design of heavy and light chains of bifunctional antibodies**

| Bispecific antibody No. | Immunoglobulin moiety | | Linker fragment | scFv moiety | | |
|---|---|---|---|---|---|---|
| | Heavy chain | Light chain | | Heavy chain variable region | Linker fragment | Light chain variable region |
| BS-PL021A | H7(hG1TM) (SEQ ID NO: 11) | L8 (SEQ ID NO: 12) | (GGGGS)3 | 14C12H1v (SEQ ID NO: 19) | (GGGGS)4 | 14C12L1(M)v (SEQ ID NO: 38) |
| BS-PL022B | H7(hG1TM) (SEQ ID NO: 11) | L8 (SEQ ID NO: 12) | (GGGGS)4 | 14C12H1v (SEQ ID NO: 19) | (GGGGS)4 | 14C12L1(M)v (SEQ ID NO: 38) |
| BS-PL023C | 14C12H1(hG1TM) (SEQ ID NO: 34) | 14C12L1 (SEQ ID NO: 25) | (GGGGS)4 | H7v (SEQ ID NO: 2) | (GGGGS)4 | L8_{V} (SEQ ID NO: 4) |
| Bs-PLV02 | 14C12H1(hG4DM) (SEQ ID NO: 32) | 14C12L1 (SEQ ID NO: 25) | (GGGGS)4 G | H7v (SEQ ID NO: 2) | (GGGGS)4 | L8_{V} (SEQ ID NO: 4) |
| Bi-PGV02 | H7(hG4DM) (SEQ ID NO: 13) | L8 (SEQ ID NO: 12) | (GGGGS)4 | 14C12H1v (SEQ ID NO: 19) | (GGGGS)4 | 14C12L1v (SEQ ID NO: 21) |

In the Table 1 above:
(1) Amino acid sequence of linker fragment (GGGGS)3:
   GGGGSGGGGSGGGGS (SEQ ID NO: 35)
   Amino acid sequence of linker fragment (GGGGS)4:
      GGGGSGGGGSGGGGSGGGGS (SEQ ID NO: 36)
   Amino acid sequence of linker fragment (GGGGS)4G:
      GGGGSGGGGSGGGGSGGGGSG (SEQ ID NO: 37)
(2) Those with "v" label at the lower right corner refer to the variable region of the corresponding heavy chain or the variable region of the corresponding light chain. For those without "v" label, the corresponding heavy or light chain is the full length comprising the constant region. The corresponding sequences described in the above preparation examples are referenced to the amino acid sequences of these variable regions or the full lengths and the nucleotide sequences encoding them.

### 2. Expression and purification of antibodies

The heavy chain cDNA sequences and the light chain cDNA sequences of Bs-PL021A, Bs-PL022B, Bs-PL023C, and Bs-PLV02 were separately cloned into pUC57simple vectors (supplied by GenScript), and plasmids pUC57simple-Bs-PL021AH/pUC57simple-Bs-PL021AL, pUC57simple-Bs-PL022BH/pUC57simple-Bs-PL022BL, pUC57simple-Bs-PL023CH/pUC57simple-Bs-PL023CL, pUC57simple-Bs-PLV02H/pUC57simple-Bs-PLV02L and
pUC57simple-Bi-PGV02/pUC57simple-Bi-PGV02 were obtained, respectively.

The plasmids pUC57simple-Bs-PL021AH/pUC57simple-Bs-PL021AL, pUC57simple-Bs-PL022BH/pUC57simple-Bs-PL022BL, pUC57simple-Bs-PL023CH/pUC57simple-Bs-PL023CL, pUC57simple-Bs-PLV02H/pUC57simple-Bs-PLV02L and pUC57simple-Bi-PGV02/pUC57simple-Bi-PGV02 were each digested (HindIII&EcoRI). The heavy and light chains isolated by electrophoresis were separately subcloned into pcDNA3.1 vectors, and recombinant plasmids were extracted to co-transfect 293F cells. After 7 days of cell culture, the culture medium was separated by high-speed centrifugation, and the supernatant was concentrated and loaded onto a HiTrap MabSelect SuRe column.

The protein was eluted in one step with an elution buffer. The target sample was isolated, and the buffer was exchanged into PBS.

### Prenaration Example 6: Prenaration of Fusion Proteins PD-1-mFc. PD-1-hFc and PDL1-hFc

The preparation of the fusion proteins PD-1-mFc, PD-1-hFc and PDL1-hFc, as well as the SDS-PAGE electrophoresis detection, were carried out by fully referring to Preparation Example 1 of Chinese Patent Publication No. CN106632674A.

The amino acid sequences and encoding nucleotide sequences of the fusion proteins PD-1-mFc, PD-1-hFc and PDL1-hFc in this preparation example are identical to those of PD-1-mFc, PD-1-hFc and PDL1-hFc in Preparation Example 1 of Chinese Patent Publication No. CN106632674A, respectively.

The fusion proteins PD-1-mFc, PD-1-hFc and PDL1-hFc were thus obtained.

### Preparation Example 7: Preparation of Human Anti-Hen Egg Lysozyme Antibody

The sequence of the human anti-hen egg lysozyme IgG (anti-HEL, or human IgG, abbreviated as hIgG) antibody was derived from the variable region sequence of the Fab F10.6.6 sequence in the study reported by Acierno et al., entitled "Affinity maturation increases the stability and plasticity of the Fv domain of anti-protein antibodies" (Acierno et al., J Mol Biol., 2007; 374(1): 130-46). The preparation method was as follows:
Nanjing Genscript Biology was entrusted to carry out codon optimization of amino acids and gene synthesis on heavy and light chain (complete sequence or variable region) genes of the human IgG antibody, and by referring to the standard technologies introduced in the "Guide to Molecular Cloning Experiments (Third Edition)" and using standard molecular cloning techniques such as PCR, enzyme digestion, DNA gel extraction, ligation transformation, colony PCR or enzyme digestion identification, the heavy and light chain genes were subcloned into the antibody heavy chain expression vector and antibody light chain expression vector of the mammalian expression system, respectively. The heavy and light chain genes of the recombinant expression vectors were further sequenced and analyzed.

After the sequences were verified to be correct, a medium or large amount of endotoxin-free expression plasmids were prepared, and the heavy and light chain expression plasmids were transiently co-transfected into HEK293 cells for recombinant antibody expression. After 7 days of culture, the cell culture medium was collected and subjected to affinity purification using an rProtein A column (GE), and the quality of the resulting antibody sample was determined using SDS-PAGE and SEC-HPLC standard analysis techniques.

### Experimental Examnle 1: Assays for Binding Activity of Anti-LAG3/Anti-PD-1 Bispecific

### Antibodies to Antigens by ELISA

### 1. Assays for the binding activity of BS-PL021A, BS-PL022B, BS-PL023C, Bs-PLV02, and 14C12H1L1(hG1TM) to antigen PD-1-mFc by indirect ELISA The procedures were as follows:

An ELISA plate was coated with human PD-1-mFc at 0.5 µg/mL and incubated at 4 °C overnight. Then the ELISA plate coated with the antigen was washed once with PBST and then blocked with a PBS solution containing 1% BSA as a blocking solution at 37 °C for 2 h. After blocking, the ELISA plate was washed 3 times with PBST. The antibodies serially diluted with PBST solution (the dilution gradients for the antibody are shown in Table 2) were added. The ELISA plate containing the test antibodies was incubated at 37 °C for 30 min and then washed 3 times with PBST. After washing, a working solution of an HRP-labeled goat anti-human IgG FC (H+L) (Jackson, Cat. No. 109-035-098) secondary antibody diluted at a ratio of 1:5000 was added, and then the plate was incubated at 37 °C for 30 min. After incubation, the plate was washed 4 times with PBST, TMB (Neogen, 308177) was added for chromogenesis in the dark for 5 min, and then a stop solution was added to terminate the chromogenic reaction. The ELISA plate was put into an ELISA plate reader immediately, and the OD value of each well in the ELISA plate was read at 450 nm. The data were analyzed and processed by SoftMax Pro 6.2.1.

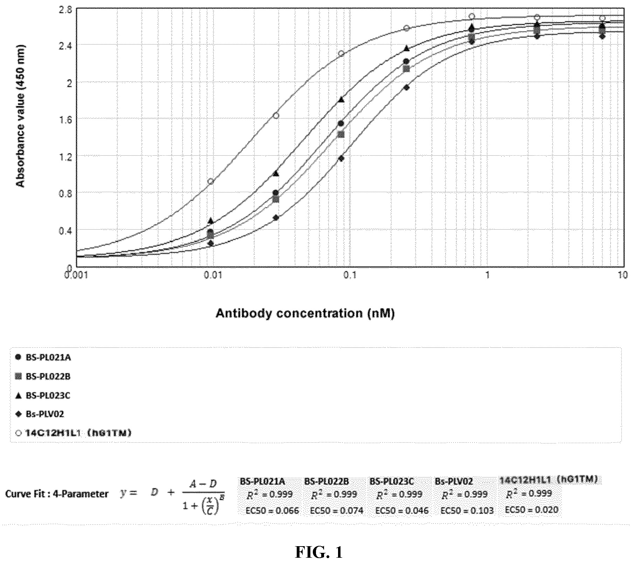

The assay results are shown in Table 2 and FIG. 1.

**Table 2: Results of assays for the binding of BS-PL021A, BS-PL022B, BS-PL023C, Bs-PLV02, and 14C12H1L1(hG1TM) to human PD-1-mFc by ELISA**

| **Antibody concentration** | **Antigen-antibody binding OD (450 nm) value** | | | | | | | | | |
|---|---|---|---|---|---|---|---|---|---|---|
| **(nM)** | **BS-PL021A** | | **BS-PL022B** | | **BS-PL023C** | | **Bs-PLV02** | | **14C12H1L1 (hG1TM)** | |
| 7.000 | 2.601 | 2.573 | 2.541 | 2.566 | 2.612 | 2.606 | 2.495 | 2.490 | 2.695 | 2.671 |
| 2.333 | 2.618 | 2.584 | 2.560 | 2.544 | 2.634 | 2.625 | 2.483 | 2.505 | 2.724 | 2.670 |
| 0.778 | 2.582 | 2.542 | 2.485 | 2.481 | 2.604 | 2.600 | 2.430 | 2.425 | 2.721 | 2.688 |
| 0.259 | 2.289 | 2.141 | 2.164 | 2.102 | 2.374 | 2.343 | 1.946 | 1.929 | 2.576 | 2.573 |
| 0.086 | 1.576 | 1.509 | 1.442 | 1.402 | 1.825 | 1.793 | 1.149 | 1.178 | 2.315 | 2.280 |
| 0.029 | 0.812 | 0.769 | 0.727 | 0.729 | 1.012 | 1.009 | 0.530 | 0.533 | 1.617 | 1.656 |
| 0.010 | 0.379 | 0.355 | 0.345 | 0.325 | 0.498 | 0.491 | 0.244 | 0.256 | 0.921 | 0.916 |
| PBST | 0.057 | 0.058 | 0.060 | 0.055 | 0.054 | 0.068 | 0.057 | 0.061 | 0.070 | 0.069 |
| EC₅₀(nM) | 0.066 | | 0.074 | | 0.046 | | 0.103 | | 0.02 | |

As can be seen from FIG. 1, BS-PL021A, BS-PL022B, BS-PL023C, Bs-PLV02, and 14C12H1L1(hG1TM) could effectively bind to the antigen human PD-1-mFc in a dose-dependent manner. By quantitative analysis of the absorbance of the bound antibodies, the binding efficiency EC₅₀ values of the antibodies BS-PL021A, BS-PL022B, BS-PL023C, Bs-PLV02, and 14C12H1L1(hG1TM) (as a control) obtained by curve fitting calculation were 0.066 nM, 0.074 nM, 0.046 nM, 0.103 nM, and 0.02 nM, respectively.

The results show that under the same experimental conditions, the binding activity of BS-PL021A, BS-PL022B and BS-PL023C to PD-1-mFc was substantially comparable to that of the positive control 14C12H1L1(hG1TM) for the same target, suggesting that BS-PL021A, BS-PL022B, BS-PL023C, and Bs-PLV02 had the activity of effectively binding to PD-1-mFc.

### 2. Assays for the binding activity of BS-PL021A, BS-PL022B, BS-PL023C, Bs-PLV02, Relatlimab, and H7L8(hG1WT) to antigen LAG3-mFc by indirect ELISA

### The procedures were as follows:

An ELISA plate was coated with human LAG3-mFc (Akeso Biopharma Inc., Batch No. 20200417) at 2 µg/mL and incubated at 4 °C overnight. Then the ELISA plate coated with the antigen was washed once with PBST and then blocked with a PBS solution containing 1% BSA as a blocking solution at 37 °C for 2 h. After blocking, the ELISA plate was washed 3 times with PBST. The antibodies serially diluted with PBST solution (the dilution gradients for the antibody are shown in Table 3) were added. The ELISA plate containing the test antibodies was incubated at 37 °C for 30 min and then washed 3 times with PBST. After washing, a working solution of an HRP-labeled goat anti-human IgG FC (H+L) (Jackson, Cat. No. 109-035-098) secondary antibody diluted at a ratio of 1:5000 was added, and then the plate was incubated at 37 °C for 30 min. After incubation, the plate was washed 4 times with PBST, TMB (Neogen, 308177) was added for chromogenesis in the dark for 5 min, and then a stop solution was added to terminate the chromogenic reaction. The ELISA plate was put into an ELISA plate reader immediately, and the OD value of each well in the ELISA plate was read at 450 nm. The data were analyzed and processed by SoftMax Pro 6.2.1.

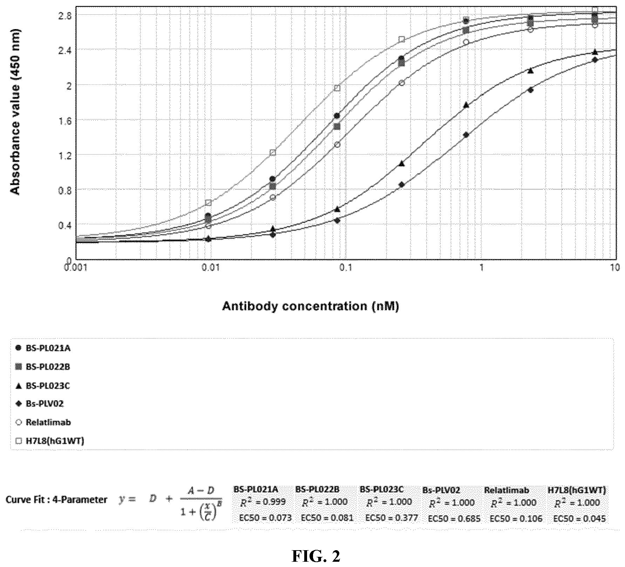

The assay results are shown in Table 3 and FIG. 2.

**Table 3: Results of assays for the binding of BS-PL021A, BS-PL022B, BS-PL023C, Bs-PLV02, Relatlimab, and H7L8(hG1WT) to LAG3-mFc by ELISA**

| **Antibody concentration** | **Antigen-antibody binding OD (450 nm) value** | | | | | | | | | | | |
|---|---|---|---|---|---|---|---|---|---|---|---|---|
| (nM) | BS-PL021A | | BS-PL022B | | BS-PL023C | | Bs-PLV02 | | Relatlimab | | H7L8(hG1WT) | |
| 7.000 | 2.843 | 2.748 | 2.743 | 2.735 | 2.388 | 2.366 | 2.311 | 2.258 | 2.703 | 2.660 | 2.852 | 2.861 |
| 2.333 | 2.757 | 2.743 | 2.717 | 2.680 | 2.194 | 2.120 | 1.970 | 1.891 | 2.620 | 2.640 | 2.746 | 2.787 |
| 0.778 | 2.766 | 2.682 | 2.622 | 2.617 | 1.746 | 1.789 | 1.431 | 1.413 | 2.478 | 2.486 | 2.687 | 2.788 |
| 0.259 | 2.342 | 2.250 | 2.246 | 2.231 | 1.126 | 1.065 | 0.860 | 0.839 | 2.006 | 2.025 | 2.466 | 2.569 |
| 0.086 | 1.707 | 1.568 | 1.525 | 1.498 | 0.550 | 0.597 | 0.438 | 0.458 | 1.294 | 1.322 | 1.895 | 2.019 |
| 0.029 | 0.960 | 0.860 | 0.820 | 0.841 | 0.352 | 0.343 | 0.275 | 0.284 | 0.703 | 0.719 | 1.176 | 1.269 |
| 0.010 | 0.503 | 0.488 | 0.453 | 0.448 | 0.247 | 0.236 | 0.238 | 0.216 | 0.394 | 0.376 | 0.620 | 0.681 |
| 0 | 0.199 | 0.204 | 0.221 | 0.181 | 0.187 | 0.186 | 0.191 | 0.211 | 0.194 | 0.187 | 0.207 | 0.221 |
| EC₅₀(nM) | 0.073 | | 0.081 | | 0.377 | | 0.685 | | 0.106 | | 0.045 | |

As can be seen from FIG. 2, BS-PL021A, BS-PL022B, BS-PL023C, Bs-PLV02, Relatlimab, and H7L8(hG1WT) could effectively bind to the antigen human LAG3-mFc in a dose-dependent manner. The absorbance intensity for each dose is shown in Table 3. By quantitative analysis of the absorbance of the bound antibodies, the binding efficiency EC₅₀ values of the antibodies BS-PL021A, BS-PL022B, BS-PL023C, Bs-PLV02, Relatlimab (as a positive control), and H7L8(hG1WT) (as a control) obtained by curve fitting calculation were 0.073 nM, 0.081 nM, 0.377 nM, 0.685 nM, 0.106 nM, and 0.045 nM, respectively.

The above experimental results show that under the same experimental conditions, BS-PL021A, BS-PL022B, and H7L8(hG1WT) had the activity of effectively binding to LAG3-mFc, and the binding activity of BS-PL021A, BS-PL022B and H7L8(hG1WT) to human LAG3-mFc was stronger than that of the positive drug Relatlimab for the same target; in particular, the binding activity of H7L8(hG1WT) to human LAG3-mFc was significantly stronger than that of the positive drug Relatlimab for the same target.

### Experimental Examnle 2: Assays for Activity of Anti-LAG3/Anti-PD-1 Bisnecific Antibodies in Competing with Human PDL1-mFc for Binding to Human PD-1-mFc-Biotin by Competitive ELISA

An ELISA plate was coated with human PDL1-mFc (PD-L1 Genbank ID: NP_054862.1, mFc SEQ ID NO:) at 2 µg/mL and incubated at 4 °C overnight. After incubation, the ELISA plate was blocked with a PBS solution containing 1% BSA at 37 °C for 2 h. After blocking, the plate was washed three times and dried. The antibody was serially diluted on a dilution plate in a 3-fold dilution ratio to achieve 7 concentrations with 80 nM as the starting concentration (at a final concentration of 40 nM), and a blank control was set. Then an equal volume of 1.2 µg/mL (at a final concentration of 0.6 µg/mL) human PD-1-mFc-Biotin solution was added and mixed well with the diluted antibody. Then the mixture was incubated at room temperature for 10 min. Then the mixture after reaction was added to the coated ELISA plate, and the ELISA plate was incubated at 37 °C for 30 min. After incubation, the plate was washed three times with PBST and dried. An SA-HRP (KPL, 14-30-00) working solution was added, and the plate was incubated at 37 °C for 30 min. After incubation, the plate was washed four times and dried. Then TMB (Neogen, 308177) was added in the dark for chromogenesis for 5 min, and then a stop solution was added to terminate the chromogenic reaction. The ELISA plate was put into an ELISA plate reader immediately, and the OD value of each well in the ELISA plate was read at 450 nm. The data were analyzed and processed by SoftMax Pro 6.2.1.

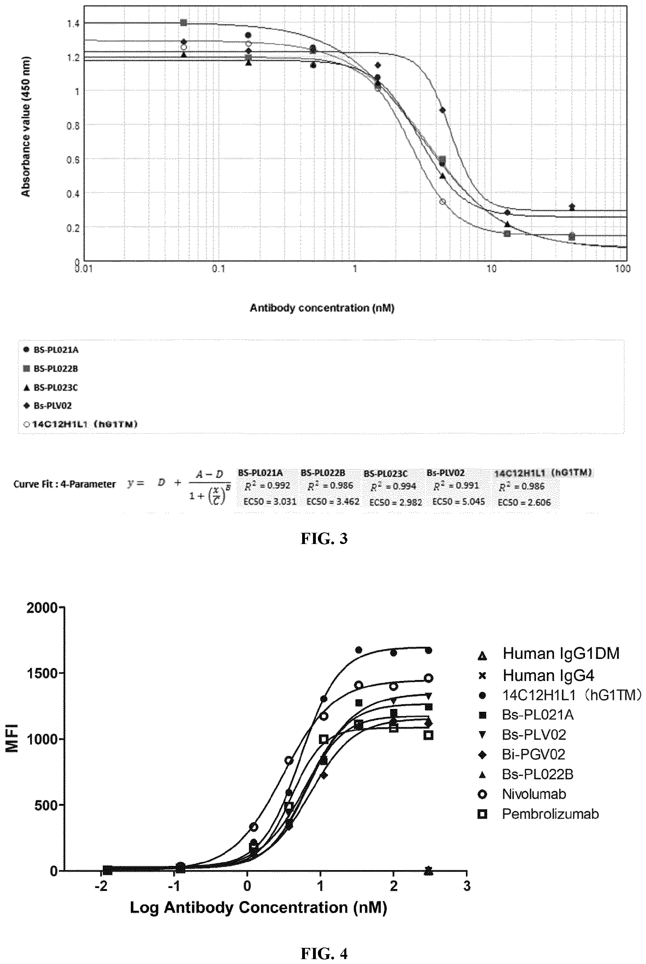

The assay results are shown in FIG. 3. The OD values for all the doses are shown in Table 4. By quantitative analysis of the absorbance intensity of the bound antibodies, the curve fitting was performed to obtain the competitive binding efficiency EC₅₀ values of the antibodies in blocking the binding of human PD-1-mFc-Biotin to its ligand human PDL1-mFc (Table 4). Table 4: Results of assays for the activity of BS-PL021A, BS-PL022B, BS-PL023C, Bs-PLV02, and 14C12H1L1(hG1TM) in competing with human PDL1-mFc for binding to human PD-1-mFc-Biotin

| **Antibody concentration** | **Results of assays for the activity of the antibodies in blocking the binding of human PD-1-mFc-Biotin to its ligand human PDL1-mFc** | | | | | | | | | |
|---|---|---|---|---|---|---|---|---|---|---|
| (nM) | BS-PL021A | | BS-PL022B | | BS-PL023C | | Bs-PLV02 | | 14C12H1L1 (hG1TM) | |
| 40.000 | 0.134 | 0.148 | 0.139 | 0.134 | 0.312 | 0.307 | 0.324 | 0.311 | 0.145 | 0.159 |
| 13.333 | 0.159 | 0.155 | 0.159 | 0.155 | 0.211 | 0.218 | 0.272 | 0.293 | 0.149 | 0.164 |
| 4.444 | 0.563 | 0.575 | 0.567 | 0.626 | 0.494 | 0.504 | 0.878 | 0.892 | 0.335 | 0.360 |
| 1.481 | 1.122 | 1.030 | 1.060 | 0.998 | 1.080 | 1.021 | 1.097 | 1.201 | 1.013 | 1.009 |
| 0.494 | 1.306 | 1.195 | 1.265 | 1.201 | 1.213 | 1.090 | 1.249 | 1.225 | 1.107 | 1.190 |
| 0.165 | 1.402 | 1.249 | 1.204 | 1.176 | 1.168 | 1.166 | 1.159 | 1.236 | 1.240 | 1.311 |
| 0.055 | 1.416 | 1.382 | 1.457 | 1.339 | 1.247 | 1.182 | 1.132 | 1.288 | 1.223 | 1.288 |
| 0 | 1.557 | 1.393 | 1.351 | 1.224 | 1.159 | 1.178 | 1.154 | 1.212 | 1.034 | 1.137 |
| EC₅₀(nM) | 3.031 | | 3.462 | | 2.982 | | 5.045 | | 2.606 | |

The results show that BS-PL021A, BS-PL022B, BS-PL023C, Bs-PLV02, and 14C12H1L1(hG1TM) (as a control) could effectively block the binding of the antigen human PD-1-mFc-Biotin to its ligand human PDL1-mFc in a dose-dependent manner, and the EC₅₀ values of BS-PL021A, BS-PL022B, BS-PL023C, Bs-PLV02, and 14C12H1L1(hG1TM) for blocking the binding of human PD-1-mFc-Biotin to its ligand human PDL1-mFc were 3.031 nM, 3.462 nM, 2.982 nM, 5.045 nM, and 2.606 nM, respectively. The efficiency of BS-PL021A, BS-PL022B, and BS-PL023C in blocking the binding of human PD-1-mFc-Biotin to its ligand human PDL1-mFc was substantially comparable to that of 14C12H1L1(hG1TM).

### Experimental Example 3: Assays for Binding Activity of Anti-LAG3/Anti-PD-1 Bispecific

### Antibodies bv FACS

### 1. Assays for the binding activity of anti-LAG3/anti-PD-1 bispecific antibodies to PD-1 on 293T-PD1 membrane surface by FACS

293T-PD1 cells in logarithmic growth phase were collected and transferred to a V-bottomed 96-well plate at 3×10⁵ cells/well. 100 µL of 1% PBSA was then added to each well, and the mixture was centrifuged at 350× g for 5 min, followed by removal of the supernatant. 100 µL of antibodies diluted with PBSA (at final concentrations of 300 nM, 100 nM, 33.3 nM, 11.1 nM, 3.7 nM, 1.23 nM, 0.123 nM, and 0.0123 nM, respectively) were added. The mixture was mixed gently and uniformly, and then incubated on ice for 1 h. 100 µL of 1% PBSA was added to each well, and the mixture was centrifuged at 350× g for 5 min, followed by removal of the supernatant. Then the plate was washed twice with 200 µL of 1% PBSA. A 400-fold diluted FITC-labeled goat anti-human IgG secondary antibody (Jackson, Cat. No. 109-095-098) was added for resuspension. The mixture was mixed well and then incubated on ice in the dark for 0.5 h. 100 µL of 1% PBSA was added to each well, and the mixture was centrifuged at 350× g for 5 min, followed by removal of the supernatant. Then the plate was washed twice with 200 µL of 1% PBSA. 400 µL of 1% PBSA was added to each well to resuspend the cell pellets, and the mixture was transferred to a flow cytometry tube for FACSCalibur assay.

The experimental results are shown in Table 5 and FIG. 4, indicating that 14C12H1L1(hG1TM), BS-PL021A, BS-PL022B, Bs-PLV02, Bi-PGV02, Nivolumab, and Pembrolizumab all could specifically bind to the PD-1 receptor on 293T-PD1 cell membrane surface.

**Table 5: Results of assays for the binding activity of 14C12H1L1(hG1TM), BS-PL021A, BS-PL022B, Bs-PLV02, Bi-PGV02, Nivolumab, and Pembrolizumab to PD-1 on 293T-PD1 cell surface by FACS**

| | BS-PL021 A | BS-PL022 B | Bs-PLV0 2 | Bi-PGV0 2 | Nivolumab | Pembrolizumab | 14C12H1L1 (hG1TM) |
|---|---|---|---|---|---|---|---|
| EC₅₀ (nM) | 6.851 | 6.066 | 6.866 | 7.206 | 3.073 | 3.970 | 5.351 |

Under the same experimental conditions, the EC₅₀ values of 14C12H1L1(hG1TM), BS-PL021A, BS-PL022B, Bs-PLV02, Bi-PGV02, Nivolumab, and Pembrolizumab for binding to 293T-PD1 cells were 5.351 nM, 6.851 nM, 6.066 nM, 6.866 nM, 7.206 nM, 3.073 nM, and 3.970 nM, respectively. The above experimental results show that under the same experimental conditions, the binding activity of 14C12H1L1(hG1TM), BS-PL021A, BS-PL022B, Bs-PLV02, and Bi-PGV02 to 293T-PD1 cells was comparable to that of the control antibodies Nivolumab and Pembrolizumab, suggesting that 14C12H1L1(hG1TM), BS-PL021A, BS-PL022B, Bs-PLV02, and Bi-PGV02 had the activity of effectively binding to PD-1 on 293T-PD1 cell membrane surface.

### 2. Assays for the binding activity of anti-LAG3/anti-PD-1 bispecific antibodies to LAG3 on 293T-LAG3 cell membrane surface by FACS

293T-LAG3 cells in logarithmic phase were digested with conventional pancreatin and transferred to a V-bottomed 96-well plate at 3×10⁵ cells/well. 100 µL of 1% PBSA was then added to each well, and the mixture was centrifuged at 350× g for 5 min, followed by removal of the supernatant. 100 µL of antibodies diluted with 1% PBSA (at final concentrations of 300 nM, 100 nM, 33.3 nM, 11.1 nM, 3.7 nM, 1.23 nM, 0.123 nM, 0.0123 nM, and 0.00123 nM, respectively) were added. The mixture was mixed well and then incubated on ice for 1 h. 100 µL of 1% PBSA was added to each well, and the mixture was centrifuged at 350× g for 5 min, followed by removal of the supernatant. Then the plate was washed twice with 200 µL of 1% PBSA. A 300-fold diluted FITC-labeled goat anti-human IgG secondary antibody (Jackson, Cat. No. 109-095-098) was added for resuspension. The mixture was mixed well and then incubated on ice in the dark for 0.5 h. 500 µL of PBSA was added to each well, and the mixture was centrifuged at 350× g for 5 min, followed by removal of the supernatant. Then the plate was washed twice with 200 µL of 1% PBSA. 300 µL of 1% PBSA was added to each well to resuspend the cell pellets, and the mixture was transferred to a flow cytometry tube for FACSCalibur assay.

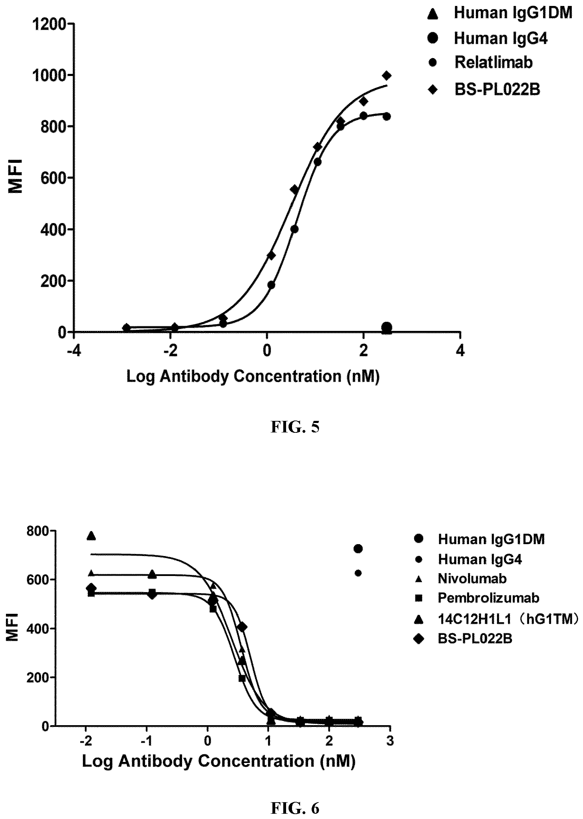

The experimental results are shown in Table 6 and FIG. 5.

**Table 6: Results of assays for the binding activity of BS-PL022B and Relatlimab to LAG3 on 293T-LAG3 cell surface by FACS**

| | Relatlimab | BS-PL022B |
|---|---|---|
| EC₅₀ (nM) | 4.113 | 3.213 |

The results show that under the same experimental conditions, the EC₅₀ values of BS-PL022B and Relatlimab for binding to LAG3 on 293T-LAG3 cell membrane surface were 3.213 nM and 4.113 nM, respectively, suggesting that the binding activity of BS-PL022B to LAG3 on 293T-LAG3 cell membrane surface was higher than that of Relatlimab.

The above experimental results show that both BS-PL022B and the positive drug Relatlimab for the same target could specifically bind to LAG-3 on 293T-LAG3 cell membrane surface in a dose-dependent manner, suggesting that BS-PL022B had the activity of effectively binding to LAG3 on 293T-LAG3 membrane surface, and the binding ability thereof was stronger than that of Relatlimab.

### Experimental Example 4: Competitive Binding of Anti-LAG3/Anti-PD-1 Bispecific

### Antibodies to Antigens on Cell Membrane Surface

### 1. Assays for the activity of anti-LAG3/anti-PD-1 bispecific antibodies in competing with PDL1 for binding to antigen PD-1 on cell membrane surface by competitive flow cytometry

293T-PD1 cells were digested conventionally and transferred to a V-bottomed 96-well plate at 3×10⁵ cells/well. 100 µL of 1% PBSA was then added to each well, and the mixture was centrifuged and washed. Corresponding serially diluted antibodies (at final concentrations of 300 nM, 100 nM, 33.3 nM, 11.1 nM, 3.7 nM, 1.23 nM, 0.123 nM, and 0.0123 nM, respectively) were each added at 100 µL per sample, and the mixture was incubated on ice for 30 min. 100 µL of PDL-1-mFc was added to each well, and the mixture was mixed well to achieve a final concentration of 20 nM, then incubated on ice for 1 h, and centrifuged at 350× g for 5 min, followed by removal of the supernatant and washing twice with 200 µL of 1% PBSA. 100 µL of a 400-fold diluted FITC goat anti-mouse IgG/IgM antibody (BD, Cat. No. 555988) was added, while 100 µL of 1% PBSA was added for a blank sample. The mixture was mixed well, incubated on ice in the dark for 30 min, washed, and centrifuged. The cells were resuspended and then transferred to a sample loading tube for testing on a flow cytometer.

The results are shown in FIG. 6, and the EC₅₀ values of the samples are shown in Table 7. By fluorescence quantitative analysis and curve fitting, the competitive binding EC₅₀ values of the antibodies Nivolumab, Pembrolizumab, 14C12H1L1(hG1TM), and BS-PL022B were calculated to be 3.608 nM, 2.769 nM, 2.511 nM, and 5.123 nM, respectively.

**Table 7: Analysis results of fluorescence intensities of Nivolumab, Pembrolizumab, 14C12H1L1(hG1TM) and BS-PL022B in competing for binding to antigen on 293T-PD-1 cell surface determined by FACS**

| | Nivolumab | Pembrolizumab | 14C12H1L1 (hG1TM) | BS-PL022B |
|---|---|---|---|---|
| EC₅₀ | 3.608 | 2.769 | 2.511 | 5.123 |

The results show that the antibody BS-PL022B could effectively block the binding of PD-L1 to PD-1 on the surface of 293T-PD1 host cells in a dose-dependent manner.

### 2. Assays for the activity of anti-LAG3/anti-PD-1 bispecific antibodies in competing with LAG3-mG1Fc for binding to antigen MHC II on cell membrane surface by competitive flow cytometry

According to the experimental design, each antibody and LAG3-mG1Fc were diluted and uniformly mixed at a 1:1 ratio, such that the final concentration of LAG3-mG1Fc (produced by Akeso Biopharma Inc., Batch No. 20190508) was 3 nM and the final concentrations of the antibody were 300 nM, 100 nM, 33.3 nM, 11.1 nM, 3.7 nM, 1.23 nM, 0.123 nM, 0.0123 nM, and 0.00123 nM. The mixture was then incubated on ice for 30 min. Raji cells (Cell Resource Center, Shanghai Institutes for Biological Sciences, Chinese Academy of Sciences, Cat. No. TCHu 44) were collected and seeded into a V-bottomed 96-well plate at 300000 cells for each sample, and then 1% PBSA was added. The mixture was centrifuged at 500× g for 5 min, followed by removal of the supernatant. The cells in each well were then resuspended in 100 µL of the antibody-protein pre-incubation solution. A blank control (cells + PBSA + PBSA), a negative control (cells + PBSA + secondary antibody), and an isotype control were designed. The system was incubated on ice in the dark for 1 h. 100 µL of 1% PBSA was then added, and the mixture was centrifuged at 500× g for 5 min, followed by removal of the supernatant. 200 µL of 1% PBSA was added to each well to resuspend the cells, and the suspension was centrifuged at 500× g for 5 min, followed by removal of the supernatant and washing once. 100 µL of APC goat anti mouse IgG secondary antibody (BioLegend, Cat. No. 405308) (diluted at a 1:300 ratio) was added to each well to resuspend the cells, while the blank control was resuspended in 100 µL of 1% PBSA. The system was incubated on ice in the dark for 30 min. 100 µL of 1% PBSA was then added, and the mixture was centrifuged at 500× g for 5 min, followed by removal of the supernatant. 200 µL of 1% PBSA was added to each well to resuspend the cells, and the suspension was centrifuged at 500× g for 5 min, followed by removal of the supernatant and washing once. 200 µL of 1% PBSA was added to each well to resuspend the cells, and the suspension was transferred to a sample loading tube for testing on a flow cytometer.

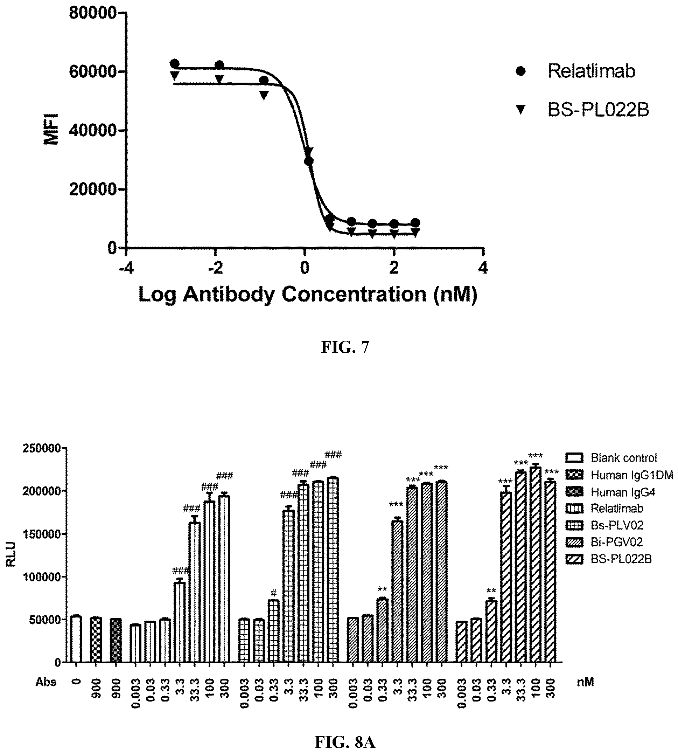

The results are shown in FIG. 7 and Table 8, and the EC₅₀ values of the samples are shown in the table. By fluorescence quantitative analysis and curve fitting, the competitive binding EC₅₀ values of the antibodies Relatlimab and BS-PL022B were calculated to be 0.9689 nM and 1.306 nM, respectively.

**Table 8: Analysis results of fluorescence intensities of Relatlimab and BS-PL022B in competing for binding to antigen on Raji cell surface determined by FACS**

| | Relatlimab | BS-PL022B |
|---|---|---|
| EC₅₀ (nM) | 0.9689 | 1.306 |
| R2 | 0.9964 | 0.9948 |

The results show that the antibody BS-PL022B could effectively block the binding of LAG-3 to MHC II on the surface of Raji host cells in a dose-dependent manner.

### Experimental Example 5: Blocking Assays for Anti-LAG3/Anti-PD-1 Bispecific Antibodies

### 1. Assays for anti-LAG3/anti-PD-1 bispecific antibodies blocking the binding of LAG3 to MHCII.

Jurkat-NFAT-PD1-LAG3 cells (constructed by Akeso Biopharma Inc., P9, viability: 97.75%) and Raji cells (Cell Resource Center, Shanghai Institutes for Biological Sciences, Chinese Academy of Sciences, Cat. No. TCHu 44) were collected and centrifuged at 110× g for 5 min, followed by removal of the supernatant. The cells were then resuspended in a 1640 medium (containing 10% FBS) and counted. The Jurkat-NFAT-PD1-LAG3 cells were seeded into a black-bottom 96-well plate (Corning, Model No. 3916) at 10×10⁴ cells/well (25 µL/well). According to the experimental design, antibodies (at final concentrations of 900 nM, 300 nM, 100 nM, 33.3 nM, 3.3 nM, 0.3 nM, 0.03 nM, and 0.003 nM, respectively) were added at 30 µL/well, and the mixture was pre-incubated at 37 °C in a 5% CO₂ incubator for 30 min. SEE (staphylococcal enterotoxin E) (at a final concentration of 0.05 ng/mL, Toxin Technology, Cat. No. ET404) and Raji cells were incubated at 37 °C in a 5% CO₂ incubator for 30 min. After incubation for 30 min, Raji cells were added into the 96-well plate at 2×10⁴ cells/well (25 µL/well) with the final volume of each well being 80 µL. The mixture was mixed well and incubated at 37 °C in a 5% CO₂ incubator for 16 h. The plate was then taken out and allowed to equilibrate to room temperature. Bright-Glo^{™} Luciferase Assay System (Promega, Cat. No. E2650) was added at 80 µL/well, and the mixture was incubated in the dark for 2 min. Then the RLU values were read.

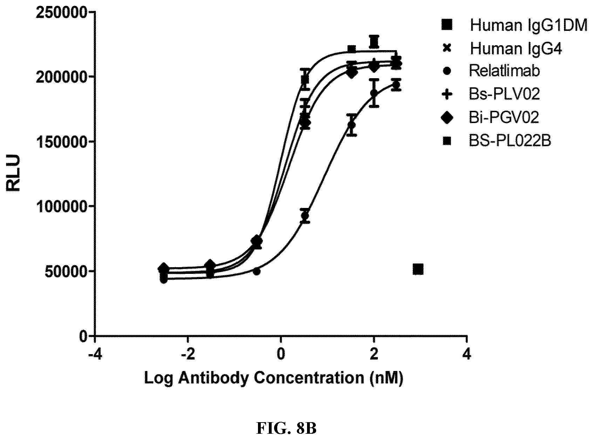

The results are shown in FIG. 8A, FIG. 8B, and Table 9.

**Table 9: Results of assays for anti-LAG3/anti-PD-1 bispecific antibodies blocking the binding of LAG3 to MHCII**

| | Relatlimab | Bs-PLV02 | Bi-PGV02 | BS-PL022B |
|---|---|---|---|---|
| EC₅₀ (nM) | 8.563 | 1.210 | 1.483 | 0.9762 |

The results show that the EC₅₀ values (nM) of Bs-PLV02, Bi-PGV02, BS-PL022B, and Relatlimab for blocking the binding of LAG3 to MHCII were 1.21 nM, 1.483 nM, 0.9762 nM, and 8.563 nM, respectively. Bs-PLV02, Bi-PGV02, and BS-PL022B had a stronger ability to block the binding of LAG3 and MHCII than the positive control antibody Relatlimab.

### 2. Assays for anti-LAG3/anti-PD-1 bispecific antibodies blocking the binding of PD-1 to PD-L1

PDL1 aAPC/CHO-K1 cells (Promega, Cat. No. J1081A) were seeded into a flat-bottom 96-well black plate (Corning, Model No. 3916) at 4×10⁴ cells/well (100 µL/well) and cultured overnight (in a medium for growth of the cells: Ham F-12 + 10% FBS). The next day, the in-plate medium was removed, and PD1 effector cells (Promega, Cat. No. J1121A) were added at 5×10⁴ cells/well (40 µL/well) (medium: 1640 + 10% FBS). Antibodies (at final concentrations of 1000 nM, 300 nM, 100 nM, 33.3 nM, 11.1 nM, 3.7 nM, 1.23 nM, 0.123 nM, and 0.0123 nM, respectively) were added at 40 µL/well, and isotype control and negative control groups were set. The final volume was 80 µL/well. The plate was placed in an incubator for incubation for 6 h. The plate was then taken out and allowed to equilibrate to room temperature. Bright-Glo^{™} Luciferase Assay System (Promega, Cat. No. E2650) was added at 80 µL/well, and the mixture was incubated in the dark for 2 min. Then the RLU values were read.

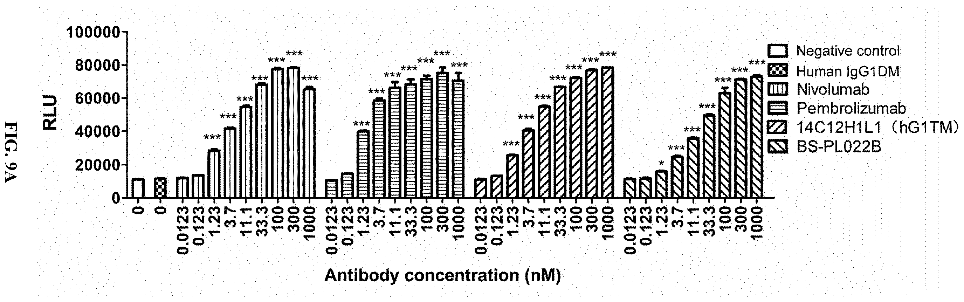

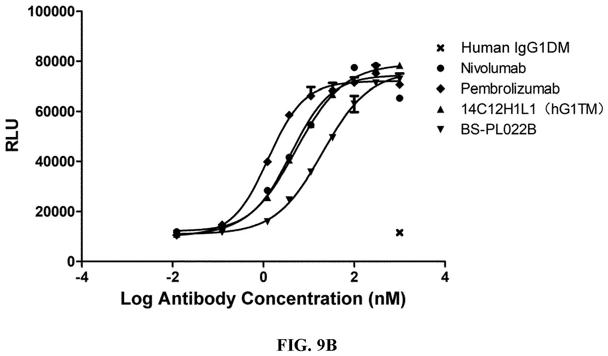

The results are shown in FIG. 9A, FIG. 9B, and Table 10.

**Table 10: Results of assays for anti-LAG3/anti-PD-1 bispecific antibodies blocking the binding of PD-1 to PD-L1**

| | Nivolumab | Pembrolizum ab | 14C12H1L1 (hG1TM) | BS-PL022B |
|---|---|---|---|---|
| EC₅₀ (nM) | 4.089 | 1.281 | 5.219 | 20.01 |

The results show that the EC₅₀ values (nM) of Nivolumab, Pembrolizumab, 14C12H1L1(hG1TM), and BS-PL022B for blocking the binding of PD-1 to PD-L1 were 4.089 nM, 1.281 nM, 5.219 nM, and 20.01nM, respectively. The results indicate that Nivolumab, Pembrolizumab, 14C12H1L1(hG1TM), and BS-PL022B all could block the binding of PD-1 to PD-L1.

### 3. Assays for anti-LAG3/anti-PD-1 bispecific antibodies simultaneously blocking the binding of LAG-3 to MHCII and PD-1 to PD-L1.

Jurkat-NFAT-PD1-LAG3 cells and Raji-PDL1 cells were collected and centrifuged at 110× g for 5 min, followed by removal of the supernatant. The cells were then resuspended in a 1640 medium (containing 10% FBS) and counted.

The Jurkat-NFAT-PD1-LAG3 cells were seeded into a black-bottom 96-well plate (Corning, Model No. 3916) at 10×10⁴ cells/well (25 µL/well). According to the experimental design, antibodies (at final concentrations of 3000 nM, 1000 nM, 300 nM, 30 nM, 3 nM, 0.3 nM, 0.03 nM, and 0.003 nM, respectively) were added at 30 µL/well, and the mixture was pre-incubated at 37 °C in a 5% CO₂ incubator for 30 min. SEE (staphylococcal enterotoxin E) (at a final concentration of 0.1 ng/mL) was added to the Raji-PDL1 cells, and then the mixture was incubated at 37 °C in a 5% CO₂ incubator for 30 min. After incubation for 30 min, the Raji-PDL1 cells were added into the 96-well plate at 3×10⁴ cells/well (25 µL/well), with the final volume of each well being 80 µL. The mixture was mixed well and incubated at 37 °C in a 5% CO₂ incubator for 15 h. The plate was then taken out and allowed to equilibrate to room temperature. Bright-Glo^{™} Luciferase Assay System (Promega, Cat. No. E2650) was added at 80 µL/well, and the mixture was incubated in the dark for 5 min. Then the RLU values were read.

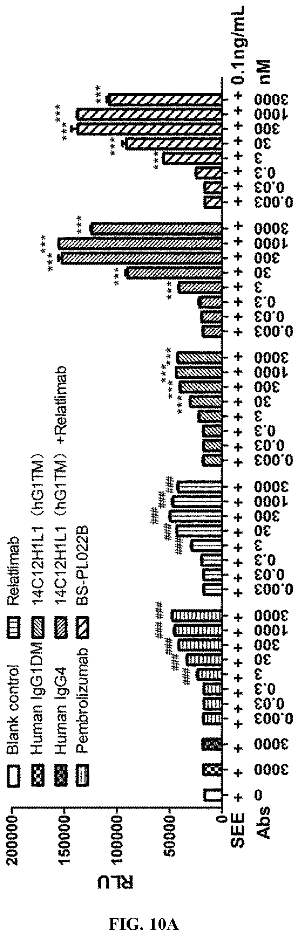

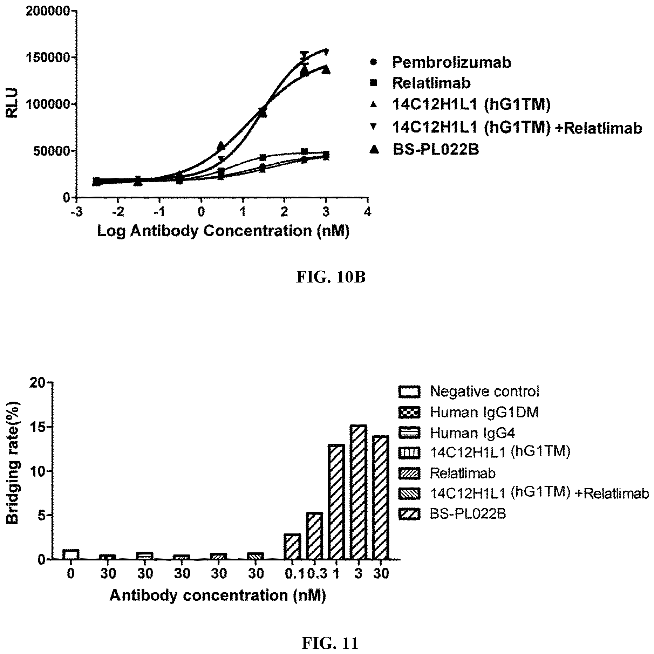

The results are shown in FIG. 10A, FIG. 10B, and Table 11.

**Table 11: Results of assays for anti-LAG3/anti-PD-1 bispecific antibodies simultaneously blocking the binding of LAG-3 to MHCII and PD-1 to PD-L1**

| | Pembrolizumab | Relatlimab | 14C12H1L1 (hG1TM) | 14C12H1L1 (hG1TM) +Relatlimab | BS-PL022B |
|---|---|---|---|---|---|
| EC₅₀ (nM) | 24.01 | 5.525 | 44.86 | 29.75 | 16.21 |

The results show that the EC₅₀ values (nM) of Pembrolizumab, Relatlimab, 14C12H1L1(hG1TM), 14C12H1L1(hG1TM) + Relatlimab, and BS-PL022B for simultaneously blocking the binding of PD1 to PD-L1 and LAG3 to MHCII were 24.01 nM, 5.525 nM, 44.86 nM, 29.75 nM, and 16.21 nM, respectively. Pembrolizumab, Relatlimab, 14C12H1L1(hG1TM), 14C12H1L1(hG1TM) + Relatlimab, and Bs-PL022B all could simultaneously block the binding of PD-1 to PD-L1 and LAG3 to MHCII, and the blocking ability of Bs-PL022B was stronger than that of other antibodies.

### Experimental Example 6: Bridging Assay for Anti-LAG3/Anti-PD-1 Bispecific Antibody

CHO-K1 cells (Cell Resource Center, Institute of Basic Medical Sciences, Chinese Academy of Medical Sciences, Cat. No. 3111C0001CCC000004), CHO-K1-PD1 cells (constructed by Akeso Biopharma Inc.), and CHO-K1-LAG3 cells (constructed by Akeso Biopharma Inc.) were digested conventionally and centrifuged at 170× g for 5 min, followed by removal of the supernatant. The cells were then resuspended in a complete medium and counted, and the cell viability was determined. The CHO-K1-PD1 cells were stained with CFSE (CFSE Cell Division Tracker Kit, Biolegend, Cat. No. 423801) (with a treatment concentration of 1 µM, 1 mL/10×10⁶ cells), the CHO-K1-LAG3 cells were stained with Far red (Thermofisher, Cat. No. C34564) (with a treatment concentration of 0.3 µM, 1 mL/10×10⁶ cells), and the CHO-K1 cells were stained with Far red or CFSE. The cells were incubated for 20 min in an incubator for staining. The staining was then stopped by adding a complete medium, and the mixture was centrifuged at 170× g for 5 min, followed by removal of the supernatant. An additional complete medium was added, and the mixture was incubated in the incubator for 10 min and centrifuged at 170× g for 5 min, followed by removal of the supernatant and washing once. The cells were then resuspended in a complete medium and counted. After staining, the CHO-K1-PD1, CHO-K1-LAG3 and CHO-K1 cells were separately transferred to a V-bottomed 96-well plate at 1.5×10⁵ cells/well, and then a buffer (PBS + 1% human serum) (the human serum was from ZhongKeChenYu Biotech Co., Ltd., Cat. No. 168014-100mL) was added. The mixture was centrifuged, followed by removal of the supernatant. According to the experimental design, antibodies (at final concentrations of 30 nM, 3 nM, 1 nM, 0.3 nM, and 0.1 nM, respectively) were added to the CHO-K1-PD1 cells at 100 µL/well, buffer or antibodies (at final concentrations of 30 nM, 3 nM, 1 nM, 0.3 nM, and 0.1 nM, respectively) were added to the CHO-K1-LAG3 cells at 100 µL/well, and buffer was added to the CHO-K1 cells at 100 µL/well. The system was then incubated on ice for 60 min. 100 µL of buffer was added, and the mixture was centrifuged at 350× g for 5 min, followed by removal of the supernatant and washing twice with 200 µL of buffer. The CHO-K1-LAG3 and CHO-K1 cells in each well were separately resuspended in 100 µL of buffer, and the suspensions were transferred to corresponding CHO-K1-PD1 sample wells at 1.5×10⁵ cells/well. The mixtures were well mixed and then incubated on ice in the dark for 40 min. 200 µL of buffer was added to each well to resuspend the cells, and the suspension was transferred to a sample loading tube for testing on a flow cytometer.

The results are shown in FIG. 11. Compared to the isotype control, the anti-LAG3/anti-PD-1 bispecific antibody could simultaneously bind to CHO-K1-PD1 and CHO-K1-LAG3 cells, bridging the two types of cells together, whereas 14C12H1L1(hG1TM) and Relatlimab, even when used in combination, did not have this effect.

### Experimental Example 7: Assay for Biological Activity of Anti-LAG3/Anti-PD-1 Bispecific

### Antibody in Promoting IFN-γ and IL-2 Secretion by Mixed Lymphocyte Reaction (MLR)

### 1. Assay for biological activity of an anti-LAG3/anti-PD-1 bispecific antibody in promoting IFN-y secretion in Raji-PDL1 mixed lymphocyte reaction system

Raji-PDL1 cells were conventionally subcultured. PBMCs were thawed, cultured in 10 mL of a 1640 complete medium, and stimulated with SEB (Staphylococcal enterotoxin B) (Dianotech, Cat. No.: S010201) at 0.5 µg/mL for two days. The Raji-PDL1 cells were treated with MMC (Stressmarq, Cat. No. SIH-246-10MG) at a final concentration of 2 µg/mL and incubated at 37 °C in a 5% CO₂ incubator for 1 h. The PBMCs stimulated with SEB for 2 days and the Raji-PDL1 cells treated with MMC for 1 h were collected and washed twice with PBS. The two types of cells were then resuspended in a complete medium, counted, separately added to a U-shaped 96-well plate (Corning, Model No. 3799) at 10×10⁴ cells/well, and co-cultured. According to the experimental design, antibodies (at final concentrations of 300 nM, 30 nM, and 3 nM, respectively) were added and co-cultured with the cells in an incubator for 3 days. After 3 days, the cells were centrifuged at 1200 rpm for 5 min, and the cell culture supernatant was collected and assayed for IFN-y by ELISA.

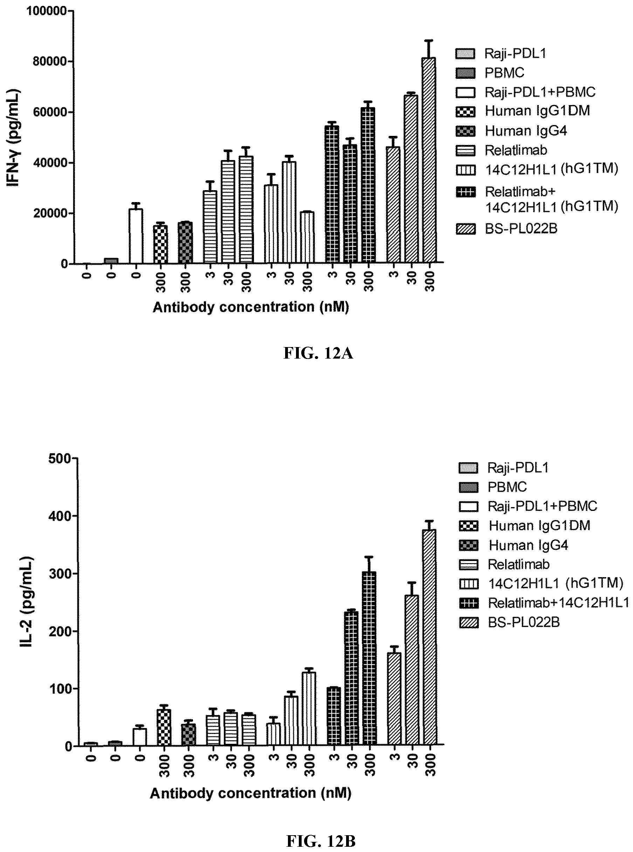

As shown in FIG. 12A, the mixed culture of human PBMCs and Raji-PDL1 cells significantly promoted the secretion of IFN-y in PBMCs, and the addition of the antibodies to the mixed culture system could significantly induce the PBMCs to further secrete IFN-γ. In terms of the level of activity in promoting IFN-y secretion, the antibody BS-PL022B was superior to the PD-1 single-target antibody 14C12H1L1 (hG1TM) and the LAG-3 single-target control antibody Relatlimab. Even compared to the combined use of 14C12H1L1 (hG1TM) and Relatlimab, BS-PL022B had better potential to promote IFN-y secretion at the two different antibody concentration levels (30 nM and 300 nM).

### 2. Assay for biological activity of an anti-LAG-3/anti-PD-1 bispecific antibody in promoting IL-2 secretion in Raji-PDL1 mixed lymphocyte reaction system

Raji-PDL1 cells were conventionally subcultured. PBMCs were thawed, cultured in 10 mL of a 1640 complete medium, and stimulated with SEB (Staphylococcal enterotoxin B) (Dianotech, Cat. No.: S010201) at 0.5 µg/mL for two days. The Raji-PDL1 cells were treated with MMC (Stressmarq, Cat. No. SIH-246-10MG) at a final concentration of 2 µg/mL and incubated at 37 °C in a 5% CO₂ incubator for 1 h. The PBMCs stimulated with SEB for 2 days and the Raji-PDL1 cells treated with MMC for 1 h were collected and washed twice with PBS. The two types of cells were then resuspended in a complete medium, counted, separately added to a U-shaped 96-well plate (Corning, Model No. 3799) at 10×10⁴ cells/well, and co-cultured. According to the experimental design, antibodies (at final concentrations of 300 nM, 30 nM, and 3 nM, respectively) were added and co-cultured with the cells for 3 days. After 3 days, the cells were centrifuged at 1200 rpm for 5 min, and the cell culture supernatant was collected and assayed for IL-2 by ELISA.

As shown in FIG. 12B, the mixed culture of human PBMCs and Raji-PDL1 cells promoted the secretion of IL-2 in PBMCs to a certain extent, and the addition of the antibodies to the mixed culture system could significantly induce the PBMCs to further secrete IL-2, exhibiting a significant dose-dependent relationship. In terms of the level of activity in promoting IL-2 secretion, compared to the PD-1 single-target antibody 14C12H1L1(hG1TM) and the LAG-3 single-target control antibody Relatlimab, BS-PL022B had better potential to promote IL-2 secretion at the three different antibody concentration levels. Even compared to the combined use of 14C12H1L1 (hG1TM) and Relatlimab, BS-PL022B had better potential to promote IL-2 secretion at the three different antibody concentration levels.

### Experimental Example 8: Assay for Affinity of BS-PL022B for Fc Receptor FcγRI

The Fc receptor FcyRI (also known as CD64) can bind to the Fc fragment of an IgG antibody and is involved in antibody-dependent cell-mediated cytotoxicity (ADCC). The binding ability of a therapeutic antibody to an Fc receptor will influence the safety and efficacy of the antibody. In this experiment, the affinity constants of BS-PL022B to FcyRI were determined using a Fortebio Octet system to evaluate the ADCC activity of the antibodies. The method for determining the affinity constants of the corresponding antibodies to FcyRI by the Fortebio Octet system is briefly described as follows: The sample dilution buffer was a solution of 0.02% Tween-20 and 0.1% BSA in PBS at pH 7.4. An FcyRI solution at a concentration of 1 µg/mL (purchased from Sinobio) was added to the HIS1K sensor to immobilize FcyRI on the sensor surface for 50 s. The association and dissociation constants of the antibody to FcyRI were both determined in the buffer with an antibody concentration of 3.12-50 nM (serial two-fold dilution). The shaking speed of the sample plate was 1000 rpm, the temperature was 30 °C and the frequency was 5.0 Hz. The data were analyzed by 1:1 model fitting to obtain affinity constants.

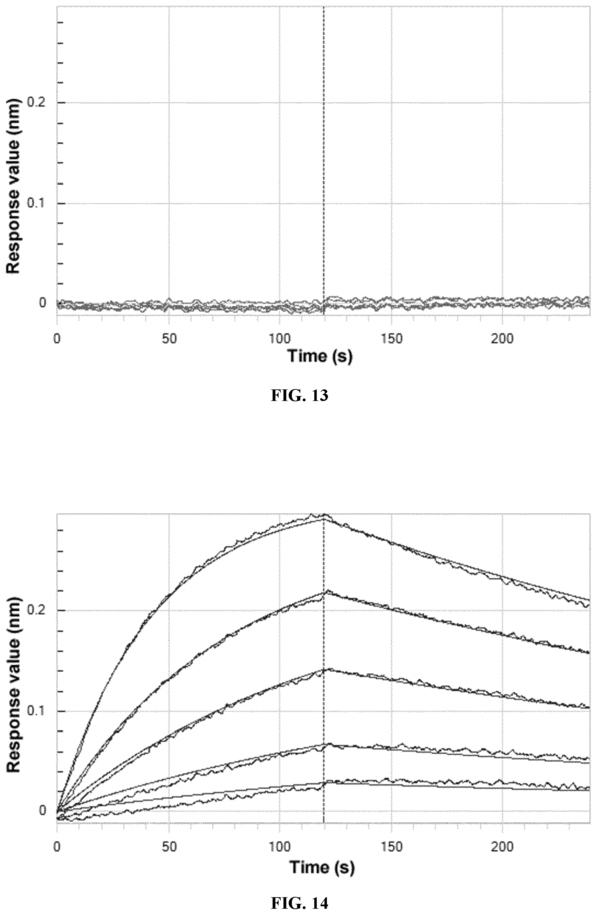

The results of the assay for the affinity constants of BS-PL022B to FcyRI are shown in Table 12 and FIGs. 13-14.

**Table 12: Kinetic parameters for binding of BS-PL022B to FcyRI**

| Antibody | K_{D} (M) | Kon (1/Ms) | SE (kon) | Kdis (1/s) | SE (kdis) | Rmax (nm) |
|---|---|---|---|---|---|---|
| BS-PL022B | N/A | N/A | N/A | N/A | N/A | N/A |
| H7L8 (hG1WT) | 6.59E-09 | 4.13E+05 | 4.38E+03 | 2.72E-03 | 2.70E-05 | 0.23-0.35 |

| | | | | | | |
|---|---|---|---|---|---|---|
| N/A indicates that the antibody had no binding or an extremely weak binding signal to the antigen, and thus the results were not analyzed and no corresponding data was obtained. | | | | | | |

The results show that H7L8(hG1WT) could bind to FcyRI with an affinity constant of 6.59E-09 M, and that BS-PL022B had no binding or an extremely weak binding signal to FcyRI, and thus the results were not analyzed and no corresponding data was obtained.

The results show that the binding activity of BS-PL022B to FcyRI was effectively eliminated.

### Experimental Example 9: Assay for Affinity of BS-PL022B for Fc Receptor FcγRIIIa and Subtype thereof

### (1) Assay for affinity constants of BS-PL022B to FcγRIIIa V158

The Fc receptor FcγRIIIa_V158 (also known as CD16a_V158) can bind to the Fc fragment of an IgG antibody and mediate ADCC effects. In this experiment, the affinity constants of BS-PL022B to FcγRIIIa_V158 were determined using a Fortebio Octet system to evaluate the ADCC activity of the antibodies.

The method for determining the affinity constants of the corresponding antibodies by the Fortebio Octet system is briefly described as follows: The sample dilution buffer was a solution of 0.02% Tween-20 and 0.1% BSA in PBS at pH 7.4. FcγRIIIa_V158 at 5 µg/mL was immobilized on the HIS1K sensor for 60 s. The sensor was equilibrated in a buffer for 60 s, and the binding of the immobilized FcγRIIIa_V158 on the sensor to the antibodies at concentrations of 31.25-500 nM (serial two-fold dilution) was determined for 60 s. The antibodies were dissociated in the buffer for 60 s. The shaking speed of the sample plate was 1000 rpm, the temperature was 30 °C and the frequency was 5.0 Hz. The data were analyzed by 1:1 model fitting to obtain affinity constants.

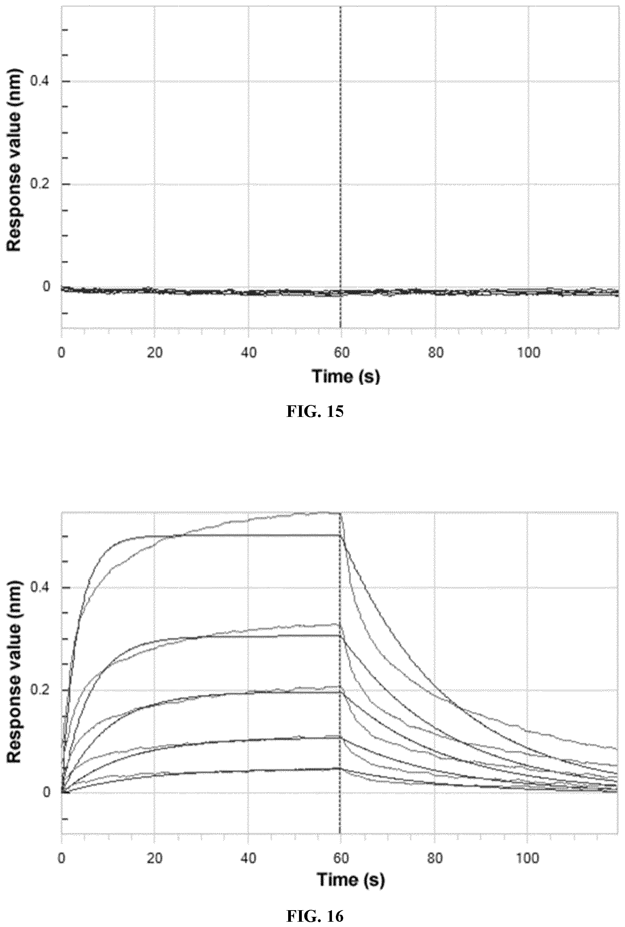

The results of the assay for the affinity constants of BS-PL022B to FcγRIIIa_V158 are shown in Table 13 and FIGs. 15-16.

**Table 13: Kinetic parameters for binding of BS-PL022B to FcγRIIIa_V158**

| Antibody | K_{D} (M) | Kon (1/Ms) | SE (kon) | Kdis (1/s) | SE (kdis) | Rmax (nm) |
|---|---|---|---|---|---|---|
| BS-PL022B | N/A | N/A | N/A | N/A | N/A | N/A |
| H7L8(hG1WT) | 8.77E-08 | 4.95E+05 | 2.36E+04 | 4.34E-02 | 7.00E-04 | 0.18-0.59 |

| | | | | | | |
|---|---|---|---|---|---|---|
| N/A indicates that the antibody had no binding or an extremely weak binding signal to the antigen, and thus the results were not analyzed and no corresponding data was obtained. | | | | | | |

The results show that H7L8(hG1WT) could bind to FcγRIIIa_V158 with an affinity constant of 8.77E-08 M, and that BS-PL022B had no binding or an extremely weak binding signal to FcγRIIIa_V158, and thus the results were not analyzed.

The results show that the binding activity of BS-PL022B to FcγRIIIa_V158 was effectively eliminated.

### (2) Assay for affinity constants of BS-PL022B to FcγRIIIa F158

The Fc receptor FcγRIIIa_F158 (also known as CD16a_F158) can bind to the Fc fragment of an IgG antibody and mediate ADCC effects. In this experiment, the affinity constants of BS-PL022B to FcγRIIIa_F158 were determined using a Fortebio Octet system to evaluate the ADCC activity of the antibodies.

The method for determining the affinity constants of TF01 to FcγRIIIa_F158 by the Fortebio Octet system is briefly described as follows: The sample dilution buffer was a solution of 0.02% Tween-20 and 0.1% BSA in PBS at pH 7.4. FcγRIIIa_F158 at 5 µg/mL was immobilized on the HIS1K sensor for 120 s. The sensor was equilibrated in a buffer for 60 s, and the binding of the immobilized FcγRIIIa_F158 on the sensor to the antibodies at concentrations of 31.25-500 nM (two-fold dilution) was determined for 60 s. The antibodies were dissociated in the buffer for 60 s. The shaking speed of the sample plate was 1000 rpm, the temperature was 30 °C and the frequency was 5.0 Hz. The data were analyzed by 1:1 model fitting to obtain affinity constants.

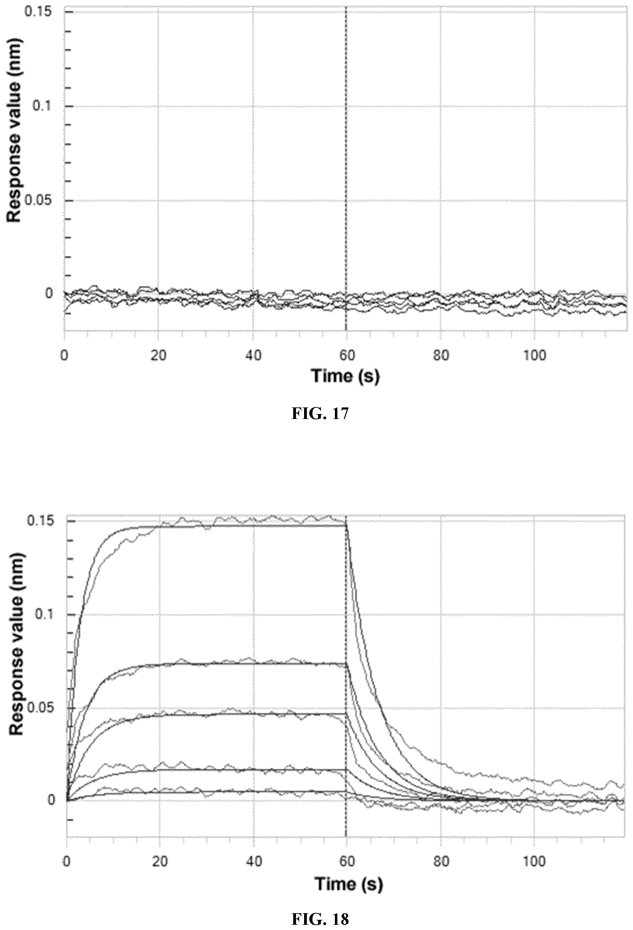

The results of the assay for the affinity constants of BS-PL022B to FcγRIIIa_F158 are shown in Table 14 and FIGs. 17-18.

**Table 14: Kinetic parameters for binding of TF01 to FcγRIIIa_F158**

| Antibody | K_{D} (M) | Kon (1/Ms) | SE (kon) | Kdis (1/s) | SE (kdis) | Rmax (nm) |
|---|---|---|---|---|---|---|
| BS-PL022B | N/A | N/A | N/A | N/A | N/A | N/A |
| H7L8(hG1WT) | 3.64E-07 | 4.15E+05 | 3.03E+04 | 1.51E-01 | 3.46E-03 | 0.06-0.25 |

| | | | | | | |
|---|---|---|---|---|---|---|
| N/A indicates that the antibody had no binding or an extremely weak binding signal to the antigen, and thus the results were not analyzed and no corresponding data was obtained. | | | | | | |

The results show that H7L8(hG1WT) could bind to FcγRIIIa_F158 with an affinity constant of 3.64E-07 M, and that BS-PL022B had no binding or an extremely weak binding signal to FcγRIIIa_F158, and thus the results were not analyzed and no corresponding data was obtained.

The results show that the binding activity of BS-PL022B to FcγRIIIa_F158 was effectively eliminated.

### Experimental Example 10: Assay for Affinity of BS-PL022B for Fc Receptor FcyRIIa and Subtype thereof

### (1) Assay for affinity constants of BS-PL022B to FcyRIIa H131

The Fc receptor FcγRIIa_H131 (also known as CD32a_H131) can bind to the Fc fragment of an IgG antibody and is involved in antibody-dependent cellular phagocytosis (ADCP) or antibody-dependent cell-mediated cytotoxicity (ADCC). The binding ability of a therapeutic antibody to an Fc receptor will influence the safety and efficacy of the antibody. In this experiment, the affinity constants of BS-PL022B to FcγRIIa_H131 were determined using a Fortebio Octet system to evaluate the binding capacity of the test antibodies to Fc receptors. The method for determining the affinity constants of BS-PL022B to FcγRIIa_H131 by the Fortebio Octet system is briefly described as follows: The sample dilution buffer was a solution of 0.02% Tween-20 and 0.1% BSA in PBS at pH 7.4. FcγRIIa_H131 at 5 µg/mL was immobilized on the NTA sensor at an immobilization height of about 1.0 nm. The sensor was equilibrated in a buffer for 60 s, and the binding of the immobilized FcγRIIa_H131 on the sensor to the antibodies at concentrations of 12.5-200 nM (serial two-fold dilution) was determined for 60 s. The antibodies were dissociated in the buffer for 60 s. The shaking speed of the sample plate was 1000 rpm, the temperature was 30 °C and the frequency was 5.0 Hz. The data were analyzed by 1:1 model fitting to obtain affinity constants.

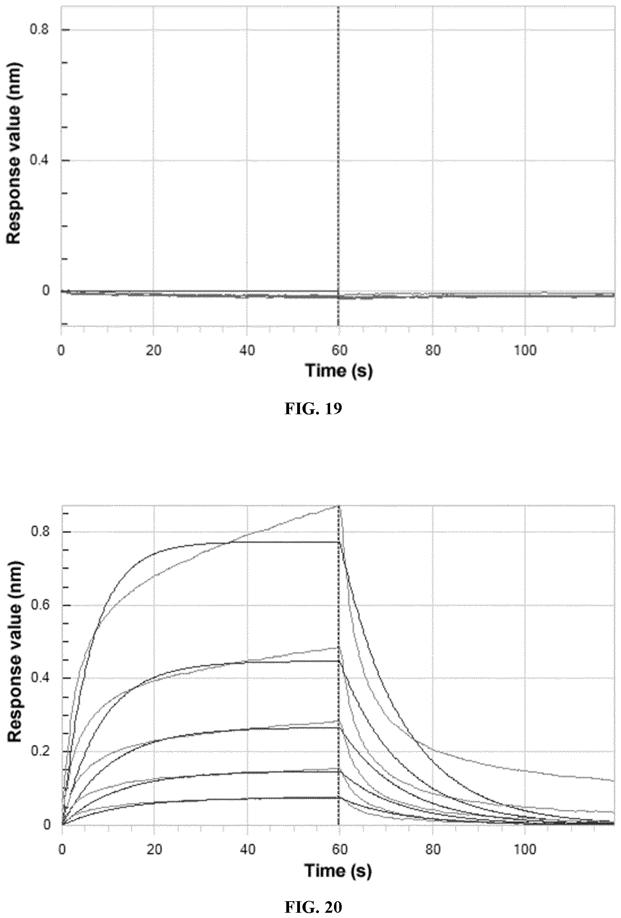

The results of the assay for the affinity constants of BS-PL022B to FcγRIIa_H131 are shown in Table 15 and FIGs. 19-20.

**Table 15: Kinetic parameters for binding of BS-PL022B to FcγRIIa_H131**

| Sample ID | K_{D} (M) | Kon (1/Ms) | SE (kon) | Kdis (1/s) | SE (kdis) | Rmax (nm) |
|---|---|---|---|---|---|---|
| BS-PL022B | N/A | N/A | N/A | N/A | N/A | N/A |
| H7L8(hG1WT) | 1.78E-07 | 4.19E+05 | 3.55E+04 | 7.44E-02 | 1.66E-03 | 1.14-1.46 |

| | | | | | | |
|---|---|---|---|---|---|---|
| N/A indicates that the antibody had no binding or an extremely weak binding signal to the antigen, and thus the results were not analyzed and no corresponding data was obtained. | | | | | | |

The results show that H7L8(hG1WT) could bind to FcγRIIa_H131 with an affinity constant of 1.78E-07 M, and that BS-PL022B had no binding or an extremely weak binding signal to FcγRIIa_H131, and thus the results were not analyzed and no corresponding data was obtained.

The results show that the binding activity of BS-PL022B to FcγRIIa_H131 was effectively eliminated.

### Experimental Examnle 11: Assay for Affinity Constants of BS-PL022B to FcγRIIb

The Fc receptor FcyRIIb (also known as CD32b) can bind to the Fc fragment of an IgG antibody. In this experiment, the affinity constants of the test antibodies to FcyRIIb were determined using a Fortebio Octet system to evaluate the binding capacity of BS-PL022B to an Fc receptor.

The method for determining the affinity constants of BS-PL022B to FcγRIIb by the Fortebio Octet system is briefly described as follows: The sample dilution buffer was a solution of 0.02% Tween-20 and 0.1% BSA in PBS at pH 7.4. FcyRIIb at 5 µg/mL was immobilized on the NTA sensor at an immobilization height of about 1.0 nm. The sensor was equilibrated in a buffer for 60 s, and the binding of the immobilized hFCGR2B-his on the sensor to the antibodies at concentrations of 12.5-200 nM (serial two-fold dilution) was determined for 60 s. The antibodies were dissociated in the buffer for 60 s. The shaking speed of the sample plate was 1000 rpm, the temperature was 30 °C and the frequency was 5.0 Hz. The data were analyzed by 1:1 model fitting to obtain affinity constants.

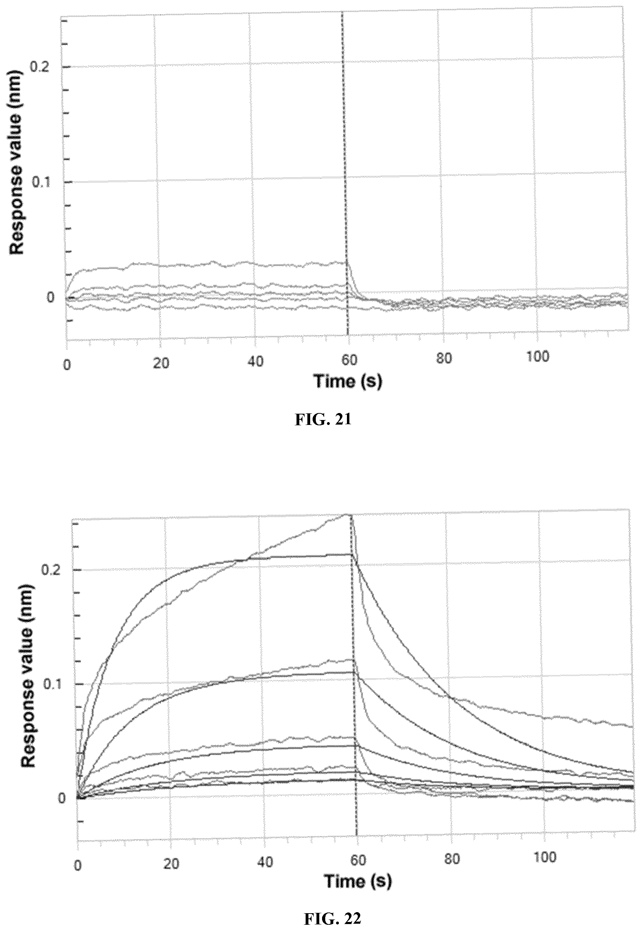

The results of the assay for the affinity constants of BS-PL022B to FcyRIIb are shown in Table 17 and FIGs. 21-22.

**Table 16: Kinetic parameters for binding of BS-PL022B to FcyRIIb**

| Antibody | K_{D} (M) | Kon (1/Ms) | SE (kon) | Kdis (1/s) | SE (kdis) | Rmax (nm) |
|---|---|---|---|---|---|---|
| BS-PL022B | N/A | N/A | N/A | N/A | N/A | N/A |
| H7L8(hG1WT) | 1.21E-07 | 3.74E+05 | 3.48E+04 | 4.53E-02 | 1.28E-03 | 0.12-0.34 |

| | | | | | | |
|---|---|---|---|---|---|---|
| N/A indicates that the antibody had no binding or an extremely weak binding signal to the antigen, and thus the results were not analyzed and no corresponding data was obtained. | | | | | | |

The results show that H7L8(hG1WT) could bind to FcyRIIb with an affinity constant of 1.21E-07 M, and that BS-PL022B had no binding or an extremely weak binding signal to FcγRIIb, and thus the results were not analyzed and no corresponding data was obtained. The results show that the binding activity of BS-PL022B to FcyRIIb was effectively eliminated.

### Experimental Example 12: Assay for Affinity of BS-PL022B for C1q

Serum complement C1q can bind to the Fc fragment of an IgG antibody and mediate CDC effects. The binding ability of a therapeutic antibody to C1q will influence the safety and efficacy of the antibody. In this experiment, the affinity constants of BS-PL022B to C1q were determined using a Fortebio Octet system to evaluate the CDC activity of the antibodies. The method for determining the affinity constants of the corresponding antibodies to C1q by the Fortebio Octet system is briefly described as follows: The sample dilution buffer was a solution of 0.02% Tween-20 and 0.1% BSA in PBS at pH 7.4. Each antibody at 50 µg/mL was immobilized on the FAB2G sensor at an immobilization height of about 2.0 nm. The sensor was equilibrated in a buffer for 60 s, and the binding of the immobilized antibody on the sensor to the antigen C1q at concentrations of 0.625-10 nM (serial two-fold dilution) was determined for 60 s. The antigen-antibody was dissociated in the buffer for 60 s. The shaking speed of the sample plate was 1000 rpm, the temperature was 30 °C and the frequency was 5.0 Hz. The data were analyzed by 1:1 model fitting to obtain affinity constants. The data acquisition software was Fortebio Data Acquisition 7.0, and the data analysis software was Fortebio Data Analysis 7.0.

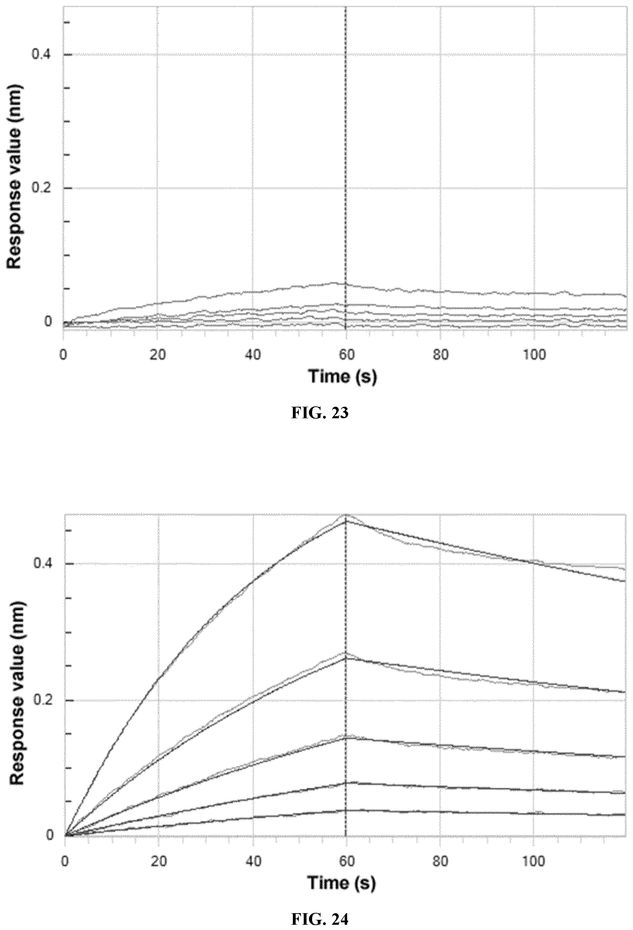

The results of the assay for the affinity constants of BS-PL022B to C1q are shown in Table 18 and FIGs. 23-24.

**Table 17: Kinetic parameters for binding of BS-PL022B to C1q**

| Antibody | K_{D} (M) | Kon (1/Ms) | SE (kon) | Kdis (1/s) | SE (kdis) | Rmax (nm) |
|---|---|---|---|---|---|---|
| BS-PL022B | N/A | N/A | N/A | N/A | N/A | N/A |
| H7L8(hG1WT) | 1.75E-09 | 2.05E+06 | 3.27E+04 | 3.58E-03 | 5.24E-05 | 0.56-0.71 |

| | | | | | | |
|---|---|---|---|---|---|---|
| N/A indicates that the antibody had no binding or an extremely weak binding signal to the antigen, and thus the results were not analyzed and no corresponding data was obtained. | | | | | | |

The results show that H7L8(hG1WT) could bind to C1q with an affinity constant of 1.75E-09 M, and that BS-PL022B had no binding or an extremely weak binding signal to C1q, and thus the results were not analyzed and no corresponding data was obtained.

The results show that the binding activity of BS-PL022B to C1q was effectively eliminated.

### Experimental Example 13: Activity of BS-PL022B in antibody-dependent cellular phagocytosis of CHO-K1-PD1-LAG3

Jurkat-NFAT-CD64-CD32R cells (constructed by Akeso Biopharma Inc.) and CHO-K1-PD1-LAG3 cells (constructed by Akeso Biopharma Inc.) were collected conventionally and centrifuged at 110× g for 5 min, followed by removal of the supernatant. The cells were then resuspended in 1640 + 4% FBS and counted. The cell viability was determined, and the cell concentration was adjusted. According to the experimental design, the test antibody was diluted to 50 nM, 5 nM and 0.5nM (the working concentrations were 10 nM, 1 nM and 0.1 nM, or 5 nM, 0.5 nM and 0.05 nM) with 1640 + 4% FBS, and the control antibody was diluted to 50 nM (the working concentration was 10 nM). The Jurkat-NFAT-CD64-CD32R cell suspension was added to a 96-well black plate at 40 µL/sample (at 40,000 cells/well). The target cells CHO-K1-PD1-LAG3 were added at 40 µL/sample (at 40,000 cells/well) to the samples already containing the Jurkat-NFAT-CD64-CD32R cells. The antibodies were added to the corresponding samples at 20 µL/well, and the mixture was mixed well. A blank control and an isotype control were also set. The plate was then incubated in an incubator for 5 h, and Bright-GloTM Luciferase Assay System (Promega, Cat. No. E2650) was added to the samples at 50 µL/well. The mixture was mixed well, and then the plate was read.

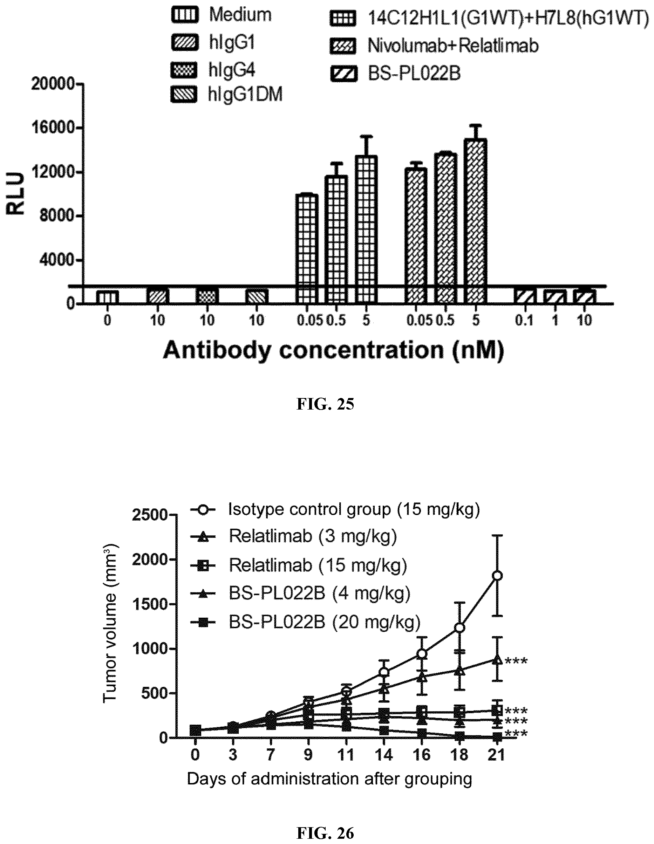

The results are shown in FIG. 25.

The results show that 14C12H1L1(G1WT) + H7L8(hG1WT) and Nivolumab + Relatlimab had ADCP effects at the same concentrations, whereas BS-PL022B did not have ADCP effects.

### Experimental Example 14: Pharmacodvnamic Evaluation of Anti-LAG3/Anti-PD-1 Bispecific Antibody in Mouse Model Subcutaneously Grafted with Tumor Cells

In order to determine the anti-tumor activity of the anti-LAG3/anti-PD-1 bispecific antibody *in vivo*, CT26 colon cancer cells (purchased from GemPharmatech Co., Ltd.) were first inoculated subcutaneously into female BALB/c-hPD1/hLAG3 mice aged 7.1-7.3 weeks (purchased from GemPharmatech Co., Ltd.) on the right hind thigh. The day of grafting was defined as D0. The route of administration was intraperitoneal injection (ip), twice per week (BIW), 6 times in total. The modeling and specific administration regimen are shown in Table 18. After the administration, the length and width of tumors in each group were measured, and the tumor volume was calculated.

**Table 18: Administration regimen of anti-LAG3/anti-PD-1 bispecific antibody for treating BALB/c-hPD1/hLAG3 mouse model grafted with CT26 colon cancer cells**

| Grouping | Number of animals | Inoculation | Administration |
|---|---|---|---|
| Isotype control, 15mg/kg | 6 | 5×10⁵ CT26 cells were inoculated subcutaneousl y into each BALB/c-hPD1/hLAG3 mouse on the right hind thigh. | The anti-HEL antibody (prepared in Preparation Example 7, Batch No. 20200704) was injected intraperitoneally at 15 mg/kg twice per week for 3 consecutive weeks. |
| BS-PL022B 4mg/kg | 6 | | BS-PL022B was injected intraperitoneally at 4 mg/kg twice per week for 3 consecutive weeks. |
| BS-PL022B 20mg/kg | 6 | | BS-PL022B was injected intraperitoneally at 20 mg/kg twice per week for 3 consecutive weeks. |
| Relatlimab 3mg/kg | 6 | | Relatlimab was injected intraperitoneally at 3 mg/kg twice per week for 3 consecutive weeks. |
| Relatlimab 15mg/kg | 6 | | Relatlimab was injected intraperitoneally at 15 mg/kg twice per week for 3 consecutive weeks. |

| | | | |
|---|---|---|---|
| Note: The doses of the isotype control at 15 mg/kg, Relatlimab at 15 mg/kg, and BS-PL022B at 20 mg/kg had the same molarity; the doses of Relatlimab at 3 mg/kg and BS-PL022B at 4 mg/kg had the same molarity. | | | |

The results are shown in FIG. 26. The results show that, compared to the isotype control antibody, both the anti-LAG3/anti-PD-1 bispecific antibody BS-PL022B and the positive control antibody Relatlimab could effectively inhibit the tumor growth in mice. The results indicate that the anti-LAG3/anti-PD-1 bispecific antibody BS-PL022B had significantly superior anti-tumor efficacy than the positive control antibody Relatlimab.

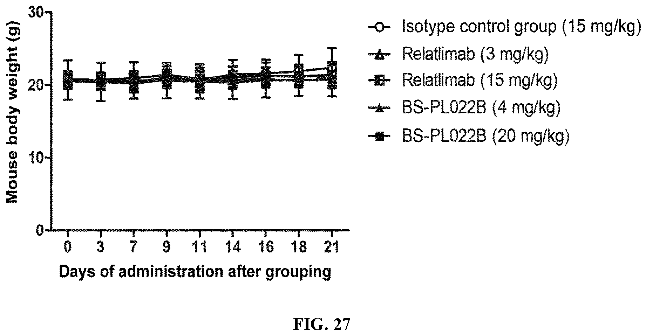

In addition, as shown in FIG. 27, the tested drug BS-PL022B was well tolerated by the tumor-bearing mice, and no impact on the body weight of the tumor-bearing mice was found in the groups.

Although specific embodiments of the present invention have been described in detail, those skilled in the art will appreciate that various modifications and substitutions can be made to those details according to all the teachings that have been disclosed, and these changes shall all fall within the protection scope of the present invention. The full scope of the present invention is given by the appended claims and any equivalent thereof.

## Claims

1. A bispecific antibody, comprising a first protein functional region and a second protein functional region, wherein
the first protein functional region targets LAG3, and
the second protein functional region targets a target other than LAG3 (e.g., PD-1),
wherein the first protein functional region is an anti-LAG3 antibody or an antigen-binding fragment thereof, comprising a heavy chain variable region and a light chain variable region,
wherein
the heavy chain variable region comprises HCDR1-HCDR3 having amino acid sequences set forth in SEQ ID NOs: 5-7, respectively, and the light chain variable region comprises LCDR1-LCDR3 having amino acid sequences set forth in SEQ ID NOs: 8-10, respectively.

2. The bispecific antibody according to claim 1, wherein
the anti-LAG3 antibody comprises a heavy chain variable region having an amino acid sequence set forth in SEQ ID NO: 2, and a light chain variable region having an amino acid sequence set forth in SEQ ID NO: 4.

3. The bispecific antibody according to any one of claims 1 to 2, wherein the anti-LAG3 antibody or the antigen-binding fragment thereof is selected from a Fab, a Fab', an F(ab')₂, an Fd, an Fv, a dAb, a complementarity determining region fragment, a single chain fragment variable, a humanized antibody, a chimeric antibody, and a diabody.

4. The bispecific antibody according to any one of claims 1 to 3, wherein the anti-LAG3 antibody binds to human LAG3-mFc with an EC₅₀ value of less than 0.2 nM, such as less than 0.15 nM, less than 0.1 nM, less than 0.08 nM, 0.06 nM, or less than 0.05 nM, or less; preferably, the EC₅₀ value is determined by indirect ELISA.

5. The bispecific antibody according to any one of claims 1 to 4, wherein
the anti-LAG3 antibody comprises a non-CDR region derived from a species other than murine, such as from a human antibody.

6. The bispecific antibody according to any one of claims 1 to 5, wherein
the anti-LAG3 antibody comprises a constant region derived from a human antibody;
preferably, the constant region of the antibody is selected from constant regions of human IgG1, IgG2, IgG3 or IgG4.

7. The bispecific antibody according to any one of claims 1 to 6, wherein
a heavy chain constant region of the anti-LAG3 antibody is Ig gamma-1 chain C region (e.g., as set forth in SEQ ID NO: 39) or Ig gamma-4 chain C region (e.g., as set forth in SEQ ID NO: 45), and a light chain constant region of the anti-LAG3 antibody is Ig kappa chain C region (e.g., as set forth in SEQ ID NO: 40).

8. The bispecific antibody according to any one of claims 1 to 7, wherein
the anti-LAG3 antibody is of human IgG1 subtype,
wherein according to the EU numbering system, the heavy chain constant region of the antibody has the following mutations:
L234A and L235A;
L234A and G237A;
L235A and G237A;
or
L234A, L235A and G237A;
preferably, the anti-LAG3 antibody comprises a heavy chain having an amino acid sequence set forth in SEQ ID NO: 11, and a light chain having an amino acid sequence set forth in SEQ ID NO: 12.

9. The bispecific antibody according to any one of claims 1 to 8, wherein
the anti-LAG3 antibody is of human IgG4 subtype,
wherein according to the EU numbering system, the heavy chain constant region of the antibody has the following mutations:
F234A and L235A;
F234A and G237A;
L235A and G237A;
or
F234A, L235A and G237A;
preferably, the anti-LAG3 antibody comprises a heavy chain having an amino acid sequence set forth in SEQ ID NO: 13, and a light chain having an amino acid sequence set forth in SEQ ID NO: 12.

10. The bispecific antibody according to any one of claims 1 to 9, wherein
preferably, the bispecific antibody is in an IgG-scFv form.

11. The bispecific antibody according to any one of claims 1 to 10, wherein
the first protein functional region is an anti-LAG3 antibody, and the second protein functional region is a single chain fragment variable; or
the first protein functional region is a single chain fragment variable, and the second protein functional region is an antibody targeting a target other than LAG3.

12. The bispecific antibody according to any one of claims 1 to 11, wherein the first protein functional region and the second protein functional region are linked directly or via a linker fragment;
preferably, the linker fragment is (GGGGS)m, m being a positive integer such as 1, 2, 3, 4, 5, or 6; or
preferably, the linker fragment is (GGGGS)nG, n being a positive integer such as 1, 2, 3, 4, 5, or 6.

13. The bispecific antibody according to any one of claims 1 to 12, wherein the numbers of the first protein functional region and the second protein functional region are each independently 1, 2, or more.

14. The bispecific antibody according to any one of claims 1 to 13, wherein the single chain fragment variable is linked to the C-terminus of the heavy chain of the antibody.

15. The bispecific antibody according to any one of claims 1 to 14, comprising:
a first protein functional region targeting LAG3, and
a second protein functional region targeting PD-1,
wherein
the first protein functional region is an anti-LAG3 antibody, and the anti-LAG3 antibody is in an immunoglobulin form;
the second protein functional region is an anti-PD-1 single chain fragment variable.

16. The bispecific antibody according to claim 15, wherein the anti-PD-1 single chain fragment variable comprises a heavy chain variable region and a light chain variable region, wherein
the heavy chain variable region comprises HCDR1-HCDR3 having amino acid sequences set forth in SEQ ID NOs: 26-28, respectively; and
the light chain variable region comprises LCDR1-LCDR3 having amino acid sequences set forth in SEQ ID NOs: 29-31, respectively.

17. The bispecific antibody according to any one of claims 15 to 16, wherein in the anti-PD-1 single chain fragment variable,
the heavy chain variable region has an amino acid sequence set forth in SEQ ID NO: 15, and
the light chain variable region has an amino acid sequence set forth in SEQ ID NO: 17; or
the heavy chain variable region has an amino acid sequence set forth in SEQ ID NO: 19, and
the light chain variable region has an amino acid sequence set forth in SEQ ID NO: 21 or SEQ ID NO: 38.

18. The bispecific antibody according to any one of claims 15 to 17, wherein the heavy chain variable region and the light chain variable region in the anti-PD-1 single chain fragment variable are linked directly or via a linker fragment;
preferably, the linker fragment is (GGGGS)m, m being a positive integer such as 1, 2, 3, 4, 5, or 6; or
preferably, the linker fragment is (GGGGS)nG, n being a positive integer such as 1, 2, 3, 4, 5, or 6.

19. The bispecific antibody according to any one of claims 15 to 18, wherein
the bispecific antibody comprises:
a first protein functional region targeting LAG3, and
a second protein functional region targeting PD-1;
the number of the first protein functional region is 1, and the number of the second protein functional region is 2;
wherein the first protein functional region is an immunoglobulin, and the second protein functional region is a single chain fragment variable;
the immunoglobulin comprises a heavy chain having an amino acid sequence set forth in SEQ ID NO: 11 or SEQ ID NO: 13, and a light chain having an amino acid sequence set forth in SEQ ID NO: 12;
the single chain fragment variable comprises a heavy chain variable region having an amino acid sequence set forth in SEQ ID NO: 19, and a light chain variable region having an amino acid sequence set forth in SEQ ID NO: 21 or SEQ ID NO: 38;
the single chain fragment variable is linked to the C termini of two heavy chains of the immunoglobulin;
the first protein functional region is linked to the second protein functional region via a first linker fragment; the heavy chain variable region of the single chain fragment variable is linked to the light chain variable region of the single chain fragment variable via a second linker fragment; the first linker fragment and the second linker fragment are identical or different;
preferably, the first linker fragment and the second linker fragment each have an amino acid sequence independently selected from SEQ ID NOs: 35-37;
preferably, amino acid sequences of the first linker fragment and the second linker fragment are set forth in SEQ ID NO: 36.

20. The bispecific antibody according to any one of claims 1 to 14, comprising:
a first protein functional region targeting LAG3, and
a second protein functional region targeting PD-1,
wherein the first protein functional region is an anti-LAG3 single chain fragment variable,
the second protein functional region is an anti-PD-1 antibody, and the anti-PD-1 antibody is in an immunoglobulin form,
wherein the anti-LAG3 single chain fragment variable comprises a heavy chain variable region and a light chain variable region, wherein
the heavy chain variable region comprises HCDR1-HCDR3 having amino acid sequences set forth in SEQ ID NOs: 5-7, respectively; and
the light chain variable region comprises LCDR1-LCDR3 having amino acid sequences set forth in SEQ ID NOs: 8-10, respectively.

21. The bispecific antibody according to claim 20, wherein in the anti-LAG3 single chain fragment variable,
the heavy chain variable region has an amino acid sequence set forth in SEQ ID NO: 2, and
the light chain variable region has an amino acid sequence set forth in SEQ ID NO: 4.

22. The bispecific antibody according to any one of claims 20 to 21, wherein the heavy chain variable region and the light chain variable region in the anti-LAG3 single chain fragment variable are linked directly or via a linker fragment;
preferably, the linker fragment is (GGGGS)m, m being a positive integer such as 1, 2, 3, 4, 5, or 6; or
preferably, the linker fragment is (GGGGS)nG, n being a positive integer such as 1, 2, 3, 4, 5, or 6.

23. The bispecific antibody according to any one of claims 20 to 22, wherein the anti-PD-1 antibody comprises a heavy chain variable region and a light chain variable region, wherein
the heavy chain variable region comprises HCDR1-HCDR3 having amino acid sequences set forth in SEQ ID NOs: 26-28, respectively; and
the light chain variable region comprises LCDR1-LCDR3 having amino acid sequences set forth in SEQ ID NOs: 29-31, respectively.

24. The bispecific antibody according to any one of claims 20 to 23, wherein in the anti-PD-1 antibody,
the heavy chain variable region has an amino acid sequence set forth in SEQ ID NO: 15, and
the light chain variable region has an amino acid sequence set forth in SEQ ID NO: 17; or
the heavy chain variable region has an amino acid sequence set forth in SEQ ID NO: 19, and
the light chain variable region has an amino acid sequence set forth in SEQ ID NO: 21 or SEQ ID NO: 38.

25. The bispecific antibody according to any one of claims 22 to 26, wherein a heavy chain constant region of the anti-PD-1 antibody is Ig gamma-1 chain C region (e.g., as set forth in SEQ ID NO: 39) or Ig gamma-4 chain C region (e.g., as set forth in SEQ ID NO: 45), and a light chain constant region of the anti-PD-1 antibody is Ig kappa chain C region (e.g., as set forth in SEQ ID NO: 40).

26. The bispecific antibody according to any one of claims 20 to 25, wherein
the anti-PD-1 antibody is of human IgG1 subtype,
wherein according to the EU numbering system, the anti-PD-1 antibody has the following mutations:
L234A and L235A;
L234A and G237A;
L235A and G237A;
or
L234A, L235A and G237A;
preferably, the anti-PD-1 antibody comprises a heavy chain having an amino acid sequence set forth in SEQ ID NO: 34, and a light chain having an amino acid sequence set forth in SEQ ID NO: 25.

27. The bispecific antibody according to any one of claims 20 to 25, wherein
the anti-PD-1 antibody is of human IgG4 subtype,
wherein according to the EU numbering system, the anti-PD-1 antibody has the following mutations:
F234A and L235A;
F234A and G237A;
L235A and G237A;
or
F234A, L235A and G237A;
preferably, the anti-PD-1 antibody comprises a heavy chain having an amino acid sequence set forth in SEQ ID NO: 32, and a light chain having an amino acid sequence set forth in SEQ ID NO: 25.

28. The bispecific antibody according to any one of claims 20 to 27, wherein
the bispecific antibody comprises:
a first protein functional region targeting LAG3, and
a second protein functional region targeting PD-1;
the number of the first protein functional region is 1, and the number of the second protein functional region is 2;
wherein the first protein functional region is a single chain fragment variable, and the second protein functional region is an immunoglobulin;
the single chain fragment variable comprises a heavy chain variable region having an amino acid sequence set forth in SEQ ID NO: 2, and a light chain variable region having an amino acid sequence set forth in SEQ ID NO: 4;
the immunoglobulin comprises a heavy chain having an amino acid sequence set forth in SEQ ID NO: 34 or SEQ ID NO: 32, and a light chain having an amino acid sequence set forth in SEQ ID NO: 25;
the single chain fragment variable is linked to the C termini of two heavy chains of the immunoglobulin;
the first protein functional region is linked to the second protein functional region via a first linker fragment; the heavy chain variable region of the single chain fragment variable is linked to the light chain variable region of the single chain fragment variable via a second linker fragment; the first linker fragment and the second linker fragment are identical or different;
preferably, the first linker fragment and the second linker fragment each have an amino acid sequence independently selected from SEQ ID NOs: 35-37;
preferably, amino acid sequences of the first linker fragment and the second linker fragment are set forth in SEQ ID NO: 36;
preferably, amino acid sequences of the first linker fragment and the second linker fragment are set forth in SEQ ID NO: 37.

29. An isolated nucleic acid molecule, encoding the bispecific antibody according to any one of claims 1 to 28.

30. A recombinant vector, comprising the isolated nucleic acid molecule according to claim 29.

31. A host cell, comprising the isolated nucleic acid molecule according to claim 29 or the recombinant vector according to claim 30.

32. A method for preparing the bispecific antibody according to any one of claims 13 to 30, comprising: culturing the host cell according to claim 31 in a suitable condition, and isolating the bispecific antibody from the cell cultures.

33. A pharmaceutical composition, comprising the bispecific antibody according to any one of claims 1 to 28, wherein optionally, the pharmaceutical composition further comprises a pharmaceutically acceptable auxiliary material.

34. Use of the bispecific antibody according to any one of claims 1 to 28 in the preparation of a medicament for treating and/or preventing a tumor or anemia, wherein
preferably, the tumor is selected from one or more of ovarian cancer, esophageal cancer, melanoma, a hematological malignancy, glioblastoma, renal cell carcinoma, lung cancer, prostate cancer, bladder cancer, colon cancer, rectal cancer, liver cancer, gastrointestinal cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

35. The bispecific antibody according to any one of claims 1 to 28 for use in treating and/or preventing a tumor or anemia, wherein
preferably, the tumor is selected from one or more of ovarian cancer, esophageal cancer, melanoma, a hematological malignancy, glioblastoma, renal cell carcinoma, lung cancer, prostate cancer, bladder cancer, colon cancer, rectal cancer, liver cancer, gastrointestinal cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.

36. A method for treating and/or preventing a tumor or anemia, comprising a step of administering to a subject in need an effective amount of the bispecific antibody according to any one of claims 1 to 28, wherein
preferably, the tumor is selected from one or more of ovarian cancer, esophageal cancer, melanoma, a hematological malignancy, glioblastoma, renal cell carcinoma, lung cancer, prostate cancer, bladder cancer, colon cancer, rectal cancer, liver cancer, gastrointestinal cancer, breast cancer, brain cancer, pancreatic cancer, thyroid cancer, head and neck cancer, and kidney cancer;
preferably, the lung cancer is non-small cell lung cancer;
preferably, the hematological malignancy is leukemia;
preferably, the esophageal cancer is esophageal squamous cancer.
